# EUROPÄISCHE PATENTSCHRIFT

(11) **EP 2 203 415 B1**
(45) Veröffentlichungstag und Bekanntmachung des Hinweises auf die Patenterteilung: **26.10.2016**
(21) Anmeldenummer: 08785814.8
(22) Anmeldetag: 04.09.2008
(51) Int. Cl.: C07C 233/56, C07C 233/88, A61K 31/165, A61P 25/28, A61P 5/50, A61P 25/18

(54) **(CYCLOPROPYL-PHENYL)-PHENYL-OXALAMIDE, VERFAHREN ZU IHRER HERSTELLUNG UND IHRE VERWENDUNG ALS ARZNEIMITTEL**
(CYCLOPROPYL-PHENYL)-PHENYL-OXALAMIDES, METHOD FOR THEIR MANUFACTURE AND THEIR APPLICATION AS MEDICINES
(CYCLOPROPANE-PHÉNYL)-PHÉNYL-OXALAMIDE, SON PROCÉDÉ DE FABRICATION ET SON UTILISATION EN TANT QUE MÉDICAMENT

(30) Priorität: 21.09.2007 EP 07291131
(43) Veröffentlichungstag der Anmeldung: 07.07.2010
(73) Patentinhaber: SANOFI, 75008 Paris (FR)
(72) Erfinder: DEFOSSA, Elisabeth, 65926 Frankfurt am Main (DE); KLABUNDE, Thomas, 65926 Frankfurt am Main (DE); DIETRICH, Viktoria, 65926 Frankfurt am Main (DE); STENGELIN, Siegfried, 65926 Frankfurt am Main (DE); HASCHKE, Guido, 65926 Frankfurt am Main (DE); HERLING, Andreas, 65926 Frankfurt am Main (DE); KUHLMANN, Johanna, 65926 Frankfurt am Main (DE); BARTOSCHEK, Stefan, 65926 Frankfurt am Main (DE)
(74) Vertreter: Fischer, Hans-Jürgen
(86) Internationale Anmeldenummer: PCT/EP2008/007217
(87) Internationale Veröffentlichungsnummer: WO 2009/039942

(56) Entgegenhaltungen:
- US-A1- 2003 092 702
- US-A1- 2005 124 667

## Beschreibung

Die Erfindung betrifft (Cyclopropyl-phenyl)-phenyl-oxalamide sowie deren physiologisch verträgliche Salze.

Es sind bereits strukturähnliche Verbindungen im Stand der Technik beschrieben (siehe US2005/0124667) sowie deren Verwendung als Antithrombotika.

Der Erfindung lag die Aufgabe zugrunde, Verbindungen zur Verfügung zu stellen, die eine therapeutisch verwertbare Wirkung entfalten. Insbesondere bestand die Aufgabe darin, neue Verbindungen zu finden, die zur Behandlung von Hyperglykämie und von Diabetes geeignet sind.

Die Erfindung betrifft daher Verbindungen der Formel I, 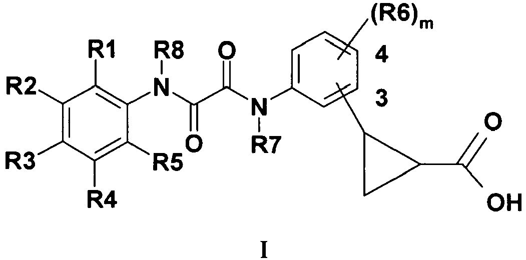 worin bedeuten
- R1, R2, R3, R4, R5: unabhängig voneinander H, F, Cl, Br, CN, CF₃, OH, OCF₃, OCHF₂, SCH₃, SCF₃, Phenyl, OPhenyl, COOH, COO-(C₁-C₆)-Alkyl, CO-(C₁-C₆)-Alkyl, (C₁-C₆)-Alkyl, O-(C₁-C₆)-Alkyl, OBn, SO₂-(C₁-C₄)-Alkyl, SO₃H, SO₂NR9R10, NR9R10 oder SO₂-N-Piperidinyl, wobei Alkyl und Phenyl ein oder mehrfach mit R12 substituiert sein können;
- R7, R8: unabhängig voneinander H oder (C₁-C₆)-Alkyl;
- m: 0, 1, 2, 3 oder 4;
- R6: OH, F, Cl, Br, CN, OCH₃, OCF₃, CH₃, CF₃, (C₁-C₆)-Alkyl oder O-(C₁-C₆)-Alkyl, wobei Alkyl ein oder mehrfach mit OH, F, Cl, Br oder CN substituiert sein kann;
- R9, R10: unabhängig voneinander H, (C₁-C₆)-Alkyl oder Phenyl, wobei Alkyl ein oder mehrfach substituiert sein kann mit F, Cl oder Br, und Phenyl ein oder mehrfach mit R6 substituiert sein kann;
- R11: F, Cl, Br, CN, OH, O-(C₁-C₆)-Alkyl, (C₁-C₆)-Alkyl, (C₂-C₆)-Alkenyl, (C₂-C₆)-Alkinyl oder NR9R10;
- R12: F, Cl, Br, CN, OH, O-(C₁-C₆)-Alkyl, (C₁-C₆)-Alkyl, (C₂-C₆)-Alkenyl, (C₂-C₆)-Alkinyl, NR9R10, COOH, COO-(C₁-C₄)-Alkyl, SCH₃, SCF₃, SO₂-(C₁-C₄)-Alkyl, SO₃H oder SO₂NR9R10;
und deren physiologisch verträgliche Salze.

Bevorzugt sind Verbindungen der Formel I, worin ein oder mehrere Reste die folgenden Bedeutungen haben:
- R1, R2, R3, R4, R5: unabhängig voneinander H, F, Cl, Br, CN, CF₃, OH, OCF₃, OCHF₂, SCH₃, SCF₃, Phenyl, OPhenyl, COOH, COO-(C₁-C₆)-Alkyl, CO-(C₁-C₆)-Alkyl, (C₁-C₆)-Alkyl, O-(C₁-C₆)-Alkyl, OBn, SO₂-(C₁-C₄)-Alkyl, SO₃H, SO₂NR9R10, NR9R10 oder SO₂-N-Piperidinyl, wobei Alkyl und Phenyl ein oder mehrfach mit R12 substituiert sein können;
- R7, R8: H;
- m: 0, 1, 2, 3 oder 4;
- R6: OH, F, Cl, Br, CN, OCH₃, OCF₃, CH₃, CF₃, (C₁-C₆)-Alkyl oder O-(C₁-C₆)-Alkyl, wobei Alkyl ein oder mehrfach mit OH, F, Cl, Br oder CN substituiert sein kann;
- R9, R10: unabhängig voneinander H, (C₁-C₆)-Alkyl oder Phenyl, wobei Alkyl ein oder mehrfach substituiert sein kann mit F, Cl oder Br, und Phenyl ein oder mehrfach mit R6 substituiert sein kann;
- R11: F, Cl, Br, CN, OH, O-(C₁-C₆)-Alkyl, (C₁-C₆)-Alkyl, (C₂-C₆)-Alkenyl, (C₂-C₆)-Alkinyl oder NR9R10;
- R12: F, Cl, Br, CN, OH, O-(C₁-C₆)-Alkyl, (C₁-C₆)-Alkyl, (C₂-C₆)-Alkenyl, (C₂-C₆)-Alkinyl, NR9R10, COOH, COO-(C₁-C₄)-Alkyl, SCH₃, SCF₃, SO₂-(C₁-C₄)-Alkyl, SO₃H oder SO₂NR9R10;
und deren physiologisch verträgliche Salze.

Besonders bevorzugt sind Verbindungen der Formel I, worin ein oder mehrere Reste die folgenden Bedeutungen haben:
- R1: H, F, Cl, Br, CN, CF₃, OH, OCF₃, OCHF₂, SCH₃, SCF₃, Phenyl, OPhenyl, COOH, COO-(C₁-C₆)-Alkyl, CO-(C₁-C₆)-Alkyl, (C₁-C₆)-Alkyl, O-(C₁-C₆)-Alkyl, OBn;
- R2: H, F, Cl, Br, CN, CF₃, OCF₃, OCHF₂, SCH₃, SCF₃, Phenyl, OPhenyl, COOH, COO-(C₁-C₆)-Alkyl, CO-(C₁-C₆)-Alkyl, (C₁-C₆)-Alkyl, O-(C₁-C₆)-Alkyl, OBn;
- R3: H, F, Cl, Br, CN, CF₃, OH, OCF₃, OCHF₂, SCH₃, SCF₃, Phenyl, OPhenyl, COOH, COO-(C₁-C₆)-Alkyl, CO-(C₁-C₆)-Alkyl, (C₁-C₆)-Alkyl, O-(C₁-C₆)-Alkyl, OBn;
- R4: H, F, Cl, Br, CN, CF₃, OCF₃, OCHF₂, SCH₃, SCF₃, Phenyl, OPhenyl, COOH, COO-(C₁-C₆)-Alkyl, CO-(C₁-C₆)-Alkyl, (C₁-C₆)-Alkyl, O-(C₁-C₆)-Alkyl, OBn;
- R5: H, F, Cl, Br, CN, CF₃, OH, OCF₃, OCHF₂, SCH₃, SCF₃, Phenyl, OPhenyl, COOH, COO-(C₁-C₆)-Alkyl, CO-(C₁-C₆)-Alkyl, (C₁-C₆)-Alkyl, O-(C₁-C₆)-Alkyl, OBn;
wobei mindestens einer der Reste R1 bis R5 eine andere Bedeutung als Wasserstoff hat;
- R7, R8: unabhängig voneinander H oder (C₁-C₆)-Alkyl;
- m: 0;
- R9, R10: unabhängig voneinander H oder (C₁-C₆)-Alkyl, wobei Alkyl ein oder mehrfach substituiert sein kann mit F, Cl oder Br;
und deren physiologisch verträgliche Salze.

Können Reste oder Substituenten mehrfach in den Verbindungen der Formeln I auftreten, so können sie alle unabhängig voneinander die angegebene Bedeutung haben und gleich oder verschieden sein.

Die Alkyl-, Alkenyl- und Alkinylreste in den Resten R1, R2, R3, R4, R5, R6, R7, R8, R9, R10, R11 und R12 können sowohl geradkettig wie verzweigt sein.

Die Erfindung betrifft Verbindungen der Formel I in Form ihrer Salze, Racemate, racemischen Mischungen und reinen Enantiomere, und deren Diastereomere und Mischungen davon.

Gegenstand der Erfindung sind weiterhin sowohl Stereoisomerengemische der Formel I, als auch die reinen Stereoisomere der Formel I, sowie Diastereomerengemische der Formel I als auch die reinen Diastereomere. Die Trennung der Gemische erfolgt z.B. auf chromatographischem Weg.

Die vorliegende Erfindung umfasst alle möglichen tautomeren Formen der Verbindungen der Formel I.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der erfindungsgemäßen Verbindungen sind Salze anorganischer Säuren, wie Salzsäure, Bromwasserstoff-, Phosphor-, Metaphosphor-, Salpeter- und Schwefelsäure sowie organischer Säuren, wie z.B. Essigsäure, Benzolsulfon-, Benzoe-, Zitronen-, Ethansulfon-, Fumar-, Glucon-, Glykol-, Isethion-, Milch-, Lactobion-, Malein-, Äpfel-, Methansulfon-, Bernstein-, p-Toluolsulfon- und Weinsäure. Geeignete pharmazeutisch verträgliche basische Salze sind Ammoniumsalze, Alkalimetallsalze (wie Natrium- und Kaliumsalze), Erdalkalisalze (wie Magnesium- und Calciumsalze), Trometamol (2-Amino-2-hydroxymethyl-1,3-propandiol), Diethanolamin, Lysin oder Ethylendiamin.

Salze mit einem nicht pharmazeutisch verträglichen Anion, wie zum Beispiel Trifluoracetat, gehören ebenfalls in den Rahmen der Erfindung als nützliche Zwischenprodukte für die Herstellung oder Reinigung pharmazeutisch verträglicher Salze und/oder für die Verwendung in nicht-therapeutischen, zum Beispiel in-vitro-Anwendungen.

Die erfindungsgemäßen Verbindungen können auch in verschiedenen polymorphen Formen vorliegen, z.B. als amorphe und kristalline polymorphe Formen. Alle polymorphen Formen der erfindungsgemäßen Verbindungen gehören in den Rahmen der Erfindung und sind ein weiterer Aspekt der Erfindung.

Nachfolgend beziehen sich alle Verweise auf "Verbindung(en) gemäß Formel I" auf Verbindung(en) der Formel I wie vorstehend beschrieben, sowie ihre Salze und Solvate wie hierin beschrieben.

Unter einem Alkylrest wird eine geradkettige oder verzweigte Kohlenwasserstoffkette verstanden, wie z.B. Methyl, Ethyl, iso-Propyl, tert.-Butyl, Hexyl. Die Alkylreste können einfach oder mehrfach wie oben beschrieben substituiert sein.

Die Erfindung umfasst auch Solvate, Hydrate und Alkoholaddukte der Verbindungen der Formel I.

Die Verbindung(en) der Formel I können auch in Kombination mit weiteren Wirkstoffen verabreicht werden.

Die Menge einer Verbindung gemäß Formel I, die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im allgemeinen liegt die Tagesdosis im Bereich von 0,3 mg bis 100 mg (typischerweise von 3 mg und 50 mg) pro Tag pro Kilogramm Körpergewicht, z.B. 3-10 mg/kg/Tag. Eine intravenöse Dosis kann z.B. im Bereich von 0,3 mg bis 1,0 mg/kg liegen, die geeigneterweise als Infusion von 10 ng bis 100 ng pro Kilogramm pro Minute verabreicht werden kann. Geeignete Infusionslösungen für diese Zwecke können z.B. von 0,1 ng bis 100 mg, typischerweise von 1 ng bis 100 mg pro Milliliter, enthalten. Einzeldosen können z.B. von 1 mg bis 10 g des Wirkstoffs enthalten. Somit können Ampullen für Injektionen beispielsweise von 1 mg bis 100 mg, und oral verabreichbare Einzeldosisformulierungen, wie zum Beispiel Tabletten oder Kapseln, können beispielsweise von 1,0 bis 1000 mg, typischerweise von 10 bis 600 mg enthalten. Zur Therapie der oben genannten Zustände können die Verbindungen gemäß Formel I selbst als Verbindung verwendet werden, vorzugsweise liegen sie jedoch mit einem verträglichen Träger in Form einer pharmazeutischen Zusammensetzung vor. Der Träger muss natürlich verträglich sein, in dem Sinne, dass er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel I. Die erfindungsgemäßen pharmazeutischen Zusammensetzungen können nach einer der bekannten pharmazeutischen Methoden hergestellt werden, die im wesentlichen darin bestehen, dass die Bestandteile mit pharmakologisch verträglichen Träger- und/oder Hilfsstoffen gemischt werden.

Erfindungsgemäße pharmazeutische Zusammensetzungen sind solche, die für orale, rektale, topische, perorale (z.B. sublinguale) und parenterale (z.B. subkutane, intramuskuläre, intradermale oder intravenöse) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel I abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Poylvinylacetatphthalat, Hydroxypropylmethylcellulosephthalat und anionische Polymere von Methacrylsäure und Methacrylsäuremethylester.

Geeignete pharmazeutische Verbindungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel I enthalten; als Pulver oder Granulate; als Lösung oder Suspension in einer wässrigen oder nicht-wässrigen Flüssigkeit; oder als eine Öl-in-Wasser- oder Wasser-in-Öl-Emulsion. Diese Zusammensetzungen können, wie bereits erwähnt, nach jeder geeigneten pharmazeutischen Methode zubereitet werden, die einen Schritt umfasst, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpresst oder geformt wird, gegebenenfalls mit einem oder mehreren zusätzlichen Bestandteilen. Gepresste Tabletten können durch tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel I mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Geeignete pharmazeutische Zusammensetzungen für die parenterale Verabreichung umfassen vorzugsweise sterile wässrige Zubereitungen einer Verbindung gemäß Formel I, die vorzugsweise isotonisch mit dem Blut des vorgesehenen Empfängers sind. Diese Zubereitungen werden vorzugsweise intravenös verabreicht, wenngleich die Verabreichung auch subkutan, intramuskulär oder intradermal als Injektion erfolgen kann. Diese Zubereitungen können vorzugsweise hergestellt werden, indem die Verbindung mit Wasser gemischt wird und die erhaltene Lösung steril und mit dem Blut isotonisch gemacht wird. Injizierbare erfindungsgemäße Zusammensetzungen enthalten im allgemeinen von 0,1 bis 5 Gew.-% der aktiven Verbindung.

Geeignete pharmazeutische Zusammensetzungen für die rektale Verabreichung liegen vorzugsweise als Einzeldosis-Zäpfchen vor. Diese können hergestellt werden, indem man eine Verbindung gemäß Formel I mit einem oder mehreren herkömmlichen festen Trägern, beispielsweise Kakaobutter, mischt und das entstehende Gemisch in Form bringt.

Geeignete pharmazeutische Zusammensetzungen für die topische Anwendung auf der Haut liegen vorzugsweise als Salbe, Creme, Lotion, Paste, Spray, Aerosol oder Öl vor. Als Träger können Vaseline, Lanolin, Polyethylenglykole, Alkohole und Kombinationen von zwei oder mehreren dieser Substanzen verwendet werden. Der Wirkstoff ist im allgemeinen in einer Konzentration von 0,1 bis 15 Gew.-% der Zusammensetzung vorhanden, beispielsweise von 0,5 bis 2%.

Auch eine transdermale Verabreichung ist möglich. Geeignete pharmazeutische Zusammensetzungen für transdermale Anwendungen können als einzelne Pflaster vorliegen, die für einen langzeitigen engen Kontakt mit der Epidermis des Patienten geeignet sind. Solche Pflaster enthalten geeigneterweise den Wirkstoff in einer gegebenenfalls gepufferten wässrigen Lösung, gelöst und/oder dispergiert in einem Haftmittel oder dispergiert in einem Polymer. Eine geeignete Wirkstoff-Konzentration beträgt ca. 1% bis 35%, vorzugsweise ca. 3% bis 15%. Als eine besondere Möglichkeit kann der Wirkstoff, wie beispielsweise in Pharmaceutical Research, 2(6): 318 (1986) beschrieben, durch Elektrotransport oder Iontophorese freigesetzt werden.

Als weitere Wirkstoffe für die Kombinationspräparate sind geeignet:
Alle Antidiabetika, die in der Roten Liste 2007, Kapitel 12 genannt sind; alle Abmagerungsmittel/Appetitzügler, die in der Roten Liste 2007, Kapitel 1 genannt sind; alle Lipidsenker, die in der Roten Liste 2007, Kapitel 58 genannt sind. Sie können mit der erfindungsgemäßen Verbindung der Formel I insbesondere zur synergistischen Wirkungsverbesserung kombiniert werden. Die Verabreichung der Wirkstoffkombination kann entweder durch getrennte Gabe der Wirkstoffe an den Patienten oder in Form von Kombinationspräparaten, worin mehrere Wirkstoffe in einer pharmazeutischen Zubereitung vorliegen, erfolgen. Die meisten der nachfolgend aufgeführten Wirkstoffe sind in USP Dictionary of USAN and International Drug Names, US Pharmacopeia, Rockville 2001, offenbart.

Antidiabetika umfassen Insulin und Insulinderivate, wie z.B. Lantus^{®} (siehe www.lantus.com) oder HMR 1964 oder Levemir® (insulin detemir) oder solche, wie sie in WO2005005477 (Novo Nordisk) beschrieben sind, schnell wirkende Insuline (siehe US 6,221,633), inhalierbare Insuline, wie z. B. Exubera ^{®} oder orale Insuline, wie z. B. IN-105 (Nobex) oder Oral-lyn ™ (Generex Biotechnology), GLP-1-Derivate und GLP-1 Agonisten wie z.B. Exenatide, Liraglutide oder diejenigen die in WO 98/08871, WO2005027978, WO2006037811, WO2006037810 von Novo Nordisk A/S, in WO 01/04156 von Zealand oder in WO 00/34331 von Beaufour-Ipsen offenbart wurden, Pramlintide Acetat (Symlin; Amylin Pharmaceuticals), BIM-51077, PC-DAC:Exendin-4 (ein Exendin-4 Analogon, welches kovalent an rekombinantes menschliches Albumin gebunden ist), Agonisten wie sie z.B. bei D. Chen et al., Proc. Natl. Acad. Sci. USA 104 (2007) 943 beschrieben sind, solche wie sie in WO2006124529 beschrieben sind, sowie oral wirksame hypoglykämische Wirkstoffe.

Antidiabetika umfassen auch Agonisten des Glukose-abhängigen insulinotropen Polypeptids (GIP) Rezeptors wie sie z.B. in WO2006121860 beschrieben sind.

Die oral wirksamen hypoglykämischen Wirkstoffe umfassen vorzugsweise Sulfonylharnstoffe,
Biguanidine,
Meglitinide,
Oxadiazolidindione,
Thiazolidindione,
Glukosidase-Inhibitoren,
Hemmstoffe der Glykogenphosphorylase,
Glukagon-Antagonisten,
Glukokinaseaktivatoren,
Inhibitoren der Fructose-1,6-bisphosphatase
Modulatoren des Glukosetransporters-4 (GLUT4),
Inhibitoren der Glutamin-Fructose-6-Phosphat-Amidotransferase (GFAT),
GLP-1-Agonisten,
Kaliumkanalöffner, wie z.B. Pinacidil, Cromakalim, Diazoxid oder solche wie sie bei R. D. Carr et al., Diabetes 52, 2003, 2513.2518, bei J. B. Hansen et al, Current Medicinal Chemistry 11, 2004, 1595-1615, bei T. M. Tagmose et al., J. Med. Chem. 47, 2004, 3202-3211 oder bei M. J. Coghlan et al., J. Med. Chem. 44, 2001, 1627-1653 beschrieben sind, oder diejenigen, die in WO 97/26265 und WO 99/03861 von Novo Nordisk A/S offenbart wurden,
Inhibitoren der Dipeptidylpeptidase-IV (DPP-IV),
Insulin-Sensitizer,
Inhibitoren von Leberenzymen, die an der Stimulation der Glukoneogenese und/oder Glykogenolyse beteiligt sind,
Modulatoren der Glukoseaufnahme, des Glukosetransports und der Glukoserückresorption, Hemmstoffe der 11ß-HSD1,
Inhibitoren der Protein-Tyrosin-Phosphatase-1B (PTP1B),
Modulatoren des natrium-abhängigen Glukosetransporters 1 oder 2 (SGLT1, SGLT2),
den Fettstoffwechsel verändernde Verbindungen wie antihyperlipidämische Wirkstoffe und antilipidämische Wirkstoffe,
Verbindungen, die die Nahrungsmitteleinnahme verringern,
Verbindungen, die die Thermogenese erhöhen,
PPAR- und RXR-Modulatoren (Retinoid X Receptor) und
Wirkstoffe, die auf den ATP-abhängigen Kaliumkanal der Betazellen wirken.

Bei einer Ausführungsform der Erfindung wird die Verbindungen der Formel I in Kombination mit einem HMGCoA-Reduktase Inhibitor (3-Hydroxy-3-methylglutaryl Coenzyme A) wie Simvastatin, Fluvastatin, Pravastatin, Lovastatin, Atorvastatin, Cerivastatin, Rosuvastatin, L-659699 verabreicht.

Bei einer Ausführungsform der Erfindung wird die Verbindung der Formel I in Kombination mit einem Cholesterinresorptionsinhibitor, wie z.B. Ezetimibe, Tiqueside, Pamaqueside, FM-VP4 (sitostanol/campesterol ascorbyl phosphat; Forbes Medi-Tech, WO2005042692, WO2005005453), MD-0727 (Microbia Inc., WO2005021497, WO2005021495) oder mit Verbindungen, wie in WO2002066464, WO2005000353 (Kotobuki Pharmaceutical Co. Ltd.) oder WO2005044256 oder WO2005062824 (Merck & Co.) oder WO2005061451 und WO2005061452 (AstraZeneca AB) und WO2006017257 (Phenomix) oder WO2005033100 (Lipideon Biotechnology AG) oder wie in WO2004097655, WO2004000805, WO2004000804, WO2004000803, WO2002050068, WO2002050060, WO2005047248, WO2006086562, WO2006102674, WO2006116499, WO2006121861, WO2006122186, WO2006122216, WO2006127893, WO2006137794, WO2006137796, WO2006137782, WO2006137793, WO2006137797, WO2006137795, WO2006137792, WO2006138163 beschrieben, verabreicht.

Bei einer Ausführungsform der Erfindung wird die Verbindung der Formel I in Kombination mit Vytorin™, einer festen Kombination von Ezetimibe mit Simvastatin, verabreicht.

Bei einer Ausführungsform der Erfindung wird die Verbindung der Formel I in Kombination mit einer festen Kombination von Ezetimibe mit Atorvastatin, verabreicht.

Bei einer Ausführungsform der Erfindung wird die Verbindung der Formel I in Kombination mit einer festen Kombination von Ezetimibe mit Fenofibrat verabreicht.

Bei einer weiteren Ausführungsform der Erfindung wird die Verbindung der Formel I in Kombination mit einer festen Kombination von Fenofibrat mit Rosuvastatin verabreicht.

Bei einer weiteren Ausführungsform der Erfindung wird die Verbindung der Formel I in Kombination mit Synordia (R), einer festen Kombination von Fenofibrat mit Metformin, verabreicht.

Bei einer Ausführungsform der Erfindung wird die Verbindung der Formel I in Kombination mit ISIS-301012, einem Antisense-Oligonukleotid, welches in der Lage ist, das Apolipoprotein B Gen zu regulieren, verabreicht.

Bei einer Ausführungsform der Erfindung wird die Verbindung der Formel I in Kombination mit einem PPAR (Peroxisome Proliferator Activated Receptor) gamma Agonisten, wie z.B. Rosiglitazon, Pioglitazon, JTT-501, Gl 262570, R-483, CS-011 (Rivoglitazon) verabreicht.

Bei einer Ausführungsform der Erfindung wird die Verbindung der Formel I in Kombination mit Competact™, einer festen Kombination von Pioglitazon Hydrochlorid mit Metformin Hydrochlorid, verabreicht.

Bei einer Ausführungsform der Erfindung wird die Verbindung der Formel I in Kombination mit Tandemact™, einer festen Kombination von Pioglitazon mit Glimeprid, verabreicht.

Bei einer weiteren Ausführungsform der Erfindung wird die Verbindung der Formel I in Kombination mit einer festen Kombination von Pioglitazon Hydrochlorid mit einem Angiotensin II Agonisten, wie z.B. TAK-536, verabreicht.

Bei einer Ausführungsform der Erfindung wird die Verbindung der Formel I in Kombination mit einem PPAR (Peroxisome Proliferator Activated Receptor) alpha Agonisten, wie z.B. GW9578, GW-590735, K-111, LY-674, KRP-101, DRF-10945, LY-518674 oder solchen wie sie in WO2001040207, WO2002096894, WO2005097076 beschrieben sind, verabreicht.

Bei einer Ausführungsform der Erfindung wird die Verbindung der Formel I in Kombination mit einem gemischten PPAR (Peroxisome Proliferator Activated Receptor) alpha/gamma Agonisten, wie z.B. Naveglitazar, LY-510929, ONO-5129, E-3030, AVE 8042, AVE 8134, AVE 0847, CKD-501 (Lobeglitazon Sulfat) oder wie in PCT/US 00/11833, PCT/US 00/11490, DE10142734.4 oder in J. P. Berger et al., TRENDS in Pharmacological Sciences 28(5), 244-251, 2005 beschrieben, verabreicht.

Bei einer Ausführungsform der Erfindung wird die Verbindung der Formel I in Kombination mit einem PPAR (Peroxisome Proliferator Activated Receptor) delta Agonisten, wie z.B. GW-501516 oder wie sie in WO2006059744, WO2006084176, WO2006029699, WO2007039172-WO2007039178 beschrieben sind, verabreicht.

Bei einer Ausführungsform wird die Verbindung der Formel I in Kombination mit Metaglidasen oder mit MBX-2044 oder anderen partiellen PPAR (Peroxisome Proliferator Activated Receptor) gamma Agonisten/Antagonisten verabreicht.

Bei einer Ausführungsform der Erfindung wird die Verbindung der Formel I in Kombination mit einem Fibrat, wie z.B. Fenofibrat, Clofibrat, Bezafibrat, verabreicht.

Bei einer Ausführungsform der Erfindung wird die Verbindung der Formel I in Kombination mit einem MTP-Inhibitor (Microsomal Triglyzerid-Transfer-Protein), wie z.B. Implitapide , BMS-201038, R-103757, AS-1552133 oder solchen wie in WO2005085226, WO2005121091, WO2006010423 beschrieben, verabreicht.

Bei einer Ausführungsform der Erfindung wird die Verbindung der Formel I in Kombination mit einem CETP-Inhibitor (Cholesterinestertransferprotein), wie z.B. Torcetrapib oder JTT-705 oder solchen wie sie in WO2006002342, WO2006010422, WO2006012093, WO2006073973, WO2006072362, WO2006097169, WO2007041494 beschrieben sind, verabreicht.

Bei einer Ausführungsform der Erfindung wird die Verbindung der Formel I in Kombination mit Gallensäureresorptionsinhibitor (siehe z.B. US 6,245,744, US 6,221,897 oder WO00/61568), wie z.B. HMR 1741 oder solchen wie in DE 10 2005 033099.1 und DE 10 2005 033100.9, WO2007009655-56 beschrieben, verabreicht.

Bei einer Ausführungsform der Erfindung wird die Verbindung der Formel I in Kombination mit einem polymeren Gallensäureadsorber, wie z.B. Cholestyramin oder Colesevelam, verabreicht.

Bei einer Ausführungsform der Erfindung wird die Verbindung der Formel I in Kombination mit einem LDL-Rezeptorinducer (Low-Density-Lipoprotein; siehe US 6,342,512), wie z.B. HMR1171, HMR1586, oder solchen wie in WO2005097738 beschrieben, verabreicht.

Bei einer Ausführungsform der Erfindung wird die Verbindung der Formel I in Kombination mit einem ABCA1 Expressionsverstäker, wie sie z.B. in WO2006072393 beschrieben, verabreicht.

Bei einer weiteren Ausführungsform der Erfindung wird die Verbindung der Formel I in Kombination mit einem RNAi Therapeutikum, welches gegen PCSK9 (Proprotein Convertase Subtilisin/Kexin Typ 9) gerichtet ist, verabreicht.

Bei einer Ausführungsform wird die Verbindung der Formel I in Kombination mit Omacor® (Omega-3-Fettsäuren; hochkonzentrierte Ethylester der Eicosapentaensäure und der Docosahexaensäure) verabreicht.

Bei einer Ausführungsform der Erfindung wird die Verbindung der Formel I in Kombination mit einem ACAT-Inhibitor (Acyl-CoA:cholesterol acyltransferase), wie z.B. Avasimibe oder SMP-797, verabreicht.

Bei einer Ausführungsform der Erfindung wird die Verbindung der Formel I in Kombination mit einem Antioxidans, wie z.B. OPC-14117, Probucol, Tocopherol, Ascorbinsäure, ß-Caroten oder Selen verabreicht.

Bei einer Ausführungsform der Erfindung wird die Verbindung der Formel I in Kombination mit einem Vitamin, wie z. B. Vitamin B6 oder Vitamin B12 verabreicht.

Bei einer Ausführungsform der Erfindung wird die Verbindung der Formel I in Kombination mit einem Lipoprotein-Lipase Modulator, wie z.B. Ibrolipim (NO-1886), verabreicht.

Bei einer Ausführungsform der Erfindung wird die Verbindung der Formel I in Kombination mit einem ATP-Citrat-Lyase Inhibitor (Adenosintriphosphat), wie z.B. SB-204990, verabreicht.

Bei einer Ausführungsform der Erfindung wird die Verbindung der Formel I in Kombination mit einem Squalen Synthetase Inhibitor, wie z.B. BMS-188494, TAK-475 oder wie in WO2005077907, JP2007022943 beschrieben, verabreicht.

Bei einer Ausführungsform der Erfindung wird die Verbindung der Formel I in Kombination mit einem Lipoprotein(a) Antagonist, wie z.B. Gemcabene (CI-1027), verabreicht.

Bei einer Ausführungsform der Erfindung wird die Verbindung der Formel I in Kombination mit einem Agonisten des GPR109A (HM74A Rezeptor Agonisten; NAR-Agonisten (Nikotinsäurerezeptoragonisten)), wie z.B. Nicotinsäure oder "extended release niacin" in Verbindung mit MK-0524A oder solchen Verbindungen, wie sie in WO2006045565, WO2006045564, WO2006069242, WO2006124490, WO2006113150, WO2007017261, WO2007017262, WO2007017265, WO2007015744, WO2007027532 beschrieben sind, verabreicht.

Bei einer anderen Ausführungsform der Erfindung wird die Verbindung der Formel I in Kombination mit einem Agonisten des GPR116, wie sie z.B. in WO2006067531, WO2006067532 beschrieben sind, verabreicht.

Bei einer Ausführungsform der Erfindung wird die Verbindung der Formel I in Kombination mit einem Lipase Inhibitor, wie z.B. Orlistat oder Cetilistat (ATL-962), verabreicht.

Bei einer Ausführungsform der Erfindung wird die Verbindung der Formel I in Kombination mit Insulin verabreicht.

Bei einer Ausführungsform wird die Verbindung der Formel I in Kombination mit einem Sulfonylharnstoff, wie z.B. Tolbutamid, Glibenclamid, Glipizid, Gliclazide oder Glimepirid verabreicht.

Bei einer Ausführungsform wird die Verbindung der Formel I in Kombination mit einer die Insulinsekretion verstärkenden Substanz, wie z. B. KCP-265 (WO2003097064), oder solchen wie sie in WO2007026761 beschrieben sind, verabreicht.

Bei einer Ausführungsform wird die Verbindung der Formel I in Kombination mit Agonisten des glucose-abhängigen insulinotropischen Rezeptors (GDIR), wie z. B. APD-668, verabreicht.

Bei einer Ausführungsform wird die Verbindung der Formel I in Kombination mit einem Biguanid, wie z.B. Metformin, verabreicht.

Bei wieder einer Ausführungsform wird die Verbindung der Formel I in Kombination mit einem Meglitinid, wie z.B. Repaglinide, Nateglinid oder Mitiglinide, verabreicht.

Bei einer weiteren Ausführungsform wird die Verbindung der Formel I mit einer Kombination von Mitiglinide mit einem Glitazon, z.B. Pioglitazon Hydrochlorid, verabreicht.

Bei einer weiteren Ausführungsform wird die Verbindung der Formel I mit einer Kombination von Mitiglinide mit einem alpha-Glukosidaseinhibitor verabreicht.

Bei einer Ausführungsform wird die Verbindung der Formel I in Kombination mit einem Thiazolidindion, wie z.B. Troglitazon, Ciglitazon, Pioglitazon, Rosiglitazon oder den in WO 97/41097 von Dr. Reddy's Research Foundation offenbarten Verbindungen, insbesondere 5-[[4-[(3,4-Dihydro-3-methyl-4-oxo-2-chinazolinylmethoxy]phenyl]methyl]-2,4-thiazolidindion, verabreicht.

Bei einer Ausführungsform wird die Verbindung der Formel I in Kombination mit einem α-Glukosidase-Inhibitor, wie z.B. Miglitol oder Acarbose, verabreicht.

Bei einer Ausführungsform wird die Verbindung der Formel I in Kombination mit einem Wirkstoff verabreicht, der auf den ATP-abhängigen Kaliumkanal der Betazellen wirkt, wie z.B. Tolbutamid, Glibenclamid, Glipizid, Glimepirid oder Repaglinid.

Bei einer Ausführungsform wird die Verbindung der Formel I in Kombination mit mehr als einer der vorstehend genannten Verbindungen, z.B. in Kombination mit einem Sulfonylharnstoff und Metformin, einem Sulfonylharnstoff und Acarbose, Repaglinid und Metformin, Insulin und einem Sulfonylharnstoff, Insulin und Metformin, Insulin und Troglitazon, Insulin und Lovastatin, etc. verabreicht.

Bei einer Ausführungsform wird die Verbindung der Formel I in Kombination mit einem Hemmstoff der Glykogenphosphorylase, wie z.B. PSN-357 oder FR-258900 oder solchen wie in WO2003084922, WO2004007455, WO2005073229-31, WO2005067932 beschrieben, verabreicht.

Bei einer Ausführungsform wird die Verbindung der Formel I in Kombination mit Glukagon-Rezeptor-Antagonisten, wie z.B. A-770077 oder NNC-25-2504 oder wie in WO2004100875, WO2005065680 beschrieben, verabreicht.

Bei einer Ausführungsform wird die Verbindung der Formel I in Kombination mit Aktivatoren der Glukokinase, wie z. B. R-1511, R-1440, LY-2121260 (WO2004063179), PSN-105, PSN-110, GKA-50 oder solchen wie sie z. B. in WO2004072031, WO2004072066, WO2005080360, WO2005044801, WO2006016194, WO2006058923, WO2006112549, WO2006125972, WO2007017549, WO2007017649, WO2007007910, WO2007007040-42, WO2007006760-61, WO2007006814, WO2007007886, WO2007028135, WO2007031739, WO2007041365, WO2007041366, WO2007037534, WO2007043638, WO2007053345, WO2007051846, WO2007051845, WO2007053765, WO2007051847 beschrieben sind, verabreicht.

Bei einer Ausführungsform wird die Verbindung der Formel I in Kombination mit einem Inhibitor der Glukoneogenese, wie z. B. FR-225654, verabreicht.

Bei einer Ausführungsform wird die Verbindung der Formel I in Kombination mit Inhibitoren der Fructose-1,6-bisphosphatase (FBPase) wie z.B. CS-917 (MB-06322) oder MB-07803 oder solchen wie sie in WO2006023515, WO2006104030, WO2007014619 beschrieben sind, verabreicht.

Bei einer Ausführungsform wird die Verbindung der Formel I in Kombination mit Modulatoren des Glukosetransporters-4 (GLUT4), wie z. B. KST-48 (D.-O. Lee et al.: Arzneim.-Forsch. Drug Res. 54 (12), 835 (2004)), verabreicht.

Bei einer Ausführungsform wird die Verbindung der Formel I in Kombination mit TNF Agonisten (Tumor necrosis factor) verabreicht.

Bei einer Ausführungsform wird die Verbindung der Formel I in Kombination mit CRF Agonisten (Corticotropin-Releasing Factor) verabreicht.

Bei einer Ausführungsform wird die Verbindung der Formel I in Kombination mit 5HT Agonisten (Serotonin Reuptake) verabreicht.

Bei einer Ausführungsform wird die Verbindung der Formel I in Kombination mit TR-β-Agonisten (Thyroid Receptor) verabreicht.

Bei einer Ausführungsform wird die Verbindung der Formel I in Kombination mit Inhibitoren der Glutamin-Fructose-6-Phosphat-Amidotransferase (GFAT), wie sie z. B. in WO2004101528 beschrieben sind, verabreicht.

Bei einer Ausführungsform wird die Verbindung der Formel I in Kombination mit Inhibitoren der Dipeptidylpeptidase-IV (DPP-IV), wie z. B. Vildagliptin (LAF-237), Sitagliptin (MK-0431), Sitagliptin Phosphat, Saxagliptin (BMS-477118), GSK-823093, PSN-9301, SYR-322, SYR-619, TA-6666, TS-021, GRC-8200, GW-825964X, KRP-104, DP-893, ABT-341, ABT-279 oder ein anderes Salz davon oder solchen Verbindungen wie sie in WO2003074500, WO2003106456, WO2004037169, WO200450658, WO2005058901, WO2005012312, WO2005/012308, WO2006039325, WO2006058064, PCT/EP2005/007821, PCT/EP2005/008005, PCT/EP2005/008002, PCT/EP2005/008004, PCT/EP2005/008283, DE 10 2005 012874.2, DE 10 2005 012873.4, JP2006160733, WO2006071752, WO2006065826, WO2006078676, WO2006073167, WO2006068163, WO2006090915, WO2006104356, WO2006127530, WO2006111261, WO2007015767, WO2007024993, WO2007029086 beschrieben sind, verabreicht.

Bei einer Ausführungsform wird die Verbindung der Formel I in Kombination mit Janumet™, einer festen Kombination von Sitagliptin Phosphat mit Metformin Hydrochlorid, verabreicht.

Bei einer Ausführungsform wird die Verbindung der Formel I in Kombination mit Hemmstoffen der 11-beta-Hydroxysteroid-Dehydrogenase- (11ß-HSD1), wie z. B. BVT-2733, JNJ-25918646, INCB-13739 oder solche, wie sie z. B. in WO200190090-94, WO200343999, WO2004112782, WO200344000, WO200344009, WO2004112779, WO2004113310, WO2004103980, WO2004112784, WO2003065983, WO2003104207, WO2003104208, WO2004106294, WO2004011410, WO2004033427, WO2004041264, WO2004037251, WO2004056744, WO2004058730, WO2004065351, WO2004089367, WO2004089380, WO2004089470-71, WO2004089896, WO2005016877, WO2005097759, WO2006010546, WO2006012227, WO2006012173, WO2006017542, WO2006034804, WO2006040329, WO2006051662, WO2006048750, WO2006049952, WO2006048331, WO2006050908, WO2006024627, WO2006040329, WO2006066109, WO2006074244, WO2006078006, WO2006106423, WO2006132436, WO2006134481, WO2006134467, WO2006135795, WO2006136502, WO2006138695, WO2006133926, WO2007003521, WO2007007688, US2007066584, WO2007047625, WO2007051811, WO2007051810 beschrieben sind, verabreicht.

Bei einer Ausführungsform wird die Verbindung der Formel I in Kombination mit Inhibitoren der Protein-Tyrosin-Phosphatase-1B (PTP1B), wie sie z. B. in WO200119830-31, WO200117516, WO2004506446, WO2005012295, WO2005116003, PCT/EP2005/005311, PCT/EP2005/005321, PCT/EP2005/007151, DE 10 2004 060542.4, WO2007009911, WO2007028145 beschrieben sind, verabreicht.

Bei einer Ausführungsform wird die Verbindung der Formel I in Kombination mit Modulatoren des natrium-abhängigen Glukosetransporters 1 oder 2 (SGLT1, SGLT2), wie z.B. KGA-2727, T-1095, SGL-0010, AVE 2268, SAR 7226 und Sergliflozin oder wie sie z. B. in WO2004007517, WO200452903, WO200452902, PCT/EP2005/005959, WO2005085237, JP2004359630, WO2005121161, WO2006018150, WO2006035796, WO2006062224, WO2006058597, WO2006073197, WO2006080577, WO2006087997, WO2006108842, WO2007000445, WO2007014895 oder von A. L. Handlon in Expert Opin. Ther. Patents (2005) 15(11), 1531-1540 beschrieben sind, verabreicht.

Bei einer Ausführungsform wird die Verbindung der Formel I in Kombination mit Modulatoren des GPR119b, wie sie z. B. in WO2004041274, beschrieben sind, verabreicht.

Bei einer Ausführungsform wird die Verbindung der Formel I in Kombination mit Modulatoren des GPR119, wie sie z. B. in WO2005061489 (PSN-632408), WO2004065380, WO2007003960-62 und WO2007003964 beschrieben sind, verabreicht.

Bei einer weiteren Ausführungsform wird die Verbindung der Formel I in Kombination mit Modulatoren des GPR120 verabreicht.

Bei einer Ausführungsform wird die Verbindung der Formel I in Kombination mit Inhibitoren der hormon-sensitiven Lipase (HSL) und/oder Phospholipasen, wie z. B. in WO2005073199, WO2006074957, WO2006087309, WO2006111321, WO2007042178 beschrieben, verabreicht.

Bei einer Ausführungsform wird die Verbindung der Formel I in Kombination mit Hemmstoffen der Acetyl-CoA Carboxylase (ACC) wie z. B. solchen wie in WO 199946262, WO200372197, WO2003072197, WO2005044814, WO2005108370, JP2006131559, WO2007011809, WO2007011811, WO2007013691 beschrieben, verabreicht.

Bei einer weiteren Ausführungsform wird die Verbindung der Formel I in Kombination mit Modulatoren der Xanthin-Oxidoreductase (XOR) verabreicht.

Bei einer Ausführungsform wird die Verbindung der Formel I in Kombination mit einem Inhibitor der Phosphoenolpyruvatcarboxykinase (PEPCK), wie z.B. solchen, wie in WO2004074288 beschrieben, verabreicht.

Bei einer Ausführungsform wird die Verbindung der Formel I in Kombination mit einem Inhibitor der Glykogen Synthase Kinase-3 beta (GSK-3 beta), wie z. B. in US2005222220, WO2005085230, PCT/EP2005/005346, WO2003078403, WO2004022544, WO2003106410, WO2005058908, US2005038023, WO2005009997, US2005026984, WO2005000836, WO2004106343, EP1460075, WO2004014910, WO2003076442, WO2005087727, WO2004046117 beschrieben, verabreicht.

Bei einer Ausführungsform wird die Verbindung der Formel I in Kombination mit einem Inhibitor der Serum/Glucocorticoid regulierten Kinase (SGK), wie z. B. in WO2006072354 beschrieben, verabreicht.

Bei einer Ausführungsform wird die Verbindung der Formel I in Kombination mit einem Agonisten des RUP3 Rezeptors, wie z. B. in WO2007035355 beschrieben, verabreicht.

Bei einer Ausführungsform wird die Verbindung der Formel I in Kombination mit einem Inhibitor der Protein Kinase C beta (PKC beta), wie z. B. Ruboxistaurin, verabreicht.

Bei einer anderen Ausführungsform wird die Verbindung der Formel I in Kombination mit einem Aktivator des Gens, welches für die Ataxia Telangiectasia Mutated (ATM) Proteinkinase kodiert, wie z. B. Chloroquin, verabreicht.

Bei einer Ausführungsform wird die Verbindung der Formel I in Kombination mit einem Endothelin-A-Rezeptor Antagonisten, wie z. B. Avosentan (SPP-301), verabreicht.

Bei einer Ausführungsform wird die Verbindung der Formel I in Kombination mit Inhibitoren der "I-kappaB kinase" (IKK Inhibitoren), wie sie z. B. in WO2001000610, WO2001030774, WO2004022553, WO2005097129 beschrieben sind, verabreicht.

Bei einer Ausführungsform wird die Verbindung der Formel I in Kombination mit Modulatoren des Glucocorticoidrezeptors (GR), wie sie z. B. in WO2005090336, WO2006071609, WO2006135826 beschrieben sind, verabreicht.

Bei einer weiteren Ausführungsform wird die Verbindung der Formel I in Kombination mit CART-Modulatoren (siehe "Cocaine-amphetamine-regulated transcript influences energy metabolism, anxiety and gastric emptying in mice" Asakawa, A. et al.: Hormone and Metabolic Research (2001), 33(9), 554-558);
NPY-Antagonisten (Neuropeptid Y) wie z.B. Naphthalin-1-sulfonsäure-{4-[(4-amino-quinazolin-2-ylamino)-methyl]-cyclohexylmethyl}-amid Hydrochlorid (CGP 71683A); NPY-5 Rezeptorantagonisten wie L-152804 oder wie sie z. B. in WO2006001318 beschrieben sind;
NPY-4-Rezeptorantagonisten wie sie z. B. in WO2007038942 beschrieben sind;
NPY-2-Rezeptorantagonisten wie sie z. B. in WO2007038943 beschrieben sind;
Peptid YY 3-36 (PYY3-36) oder analoge Verbindungen wie z. B. CJC-1682 (PYY3-36 konjugiert mit humanem Serum Albumin über Cys34) oder CJC-1643 (Derivat des PYY3-36, welches sich in vivo an Serum Albumin konjugiert) oder solche, wie sie in WO2005080424, WO2006095166 beschrieben sind;
Derivate des Peptids Obestatin wie sie WO2006096847 beschrieben sind; CB1R (Cannabinoid Rezeptor 1) Antagonisten (wie z.B. Rimonabant, SR147778, SLV-319, AVE-1625, MK-0364 oder Salze davon oder solche Verbindungen wie sie in z. B. EP 0656354, WO 00/15609, WO 02/076949, WO2005080345, WO2005080328, WO2005080343, WO2005075450, WO2005080357, WO200170700, WO2003026647-48, WO200302776, WO2003040107, WO2003007887, WO2003027069, US6,509,367, WO200132663, WO2003086288, WO2003087037, WO2004048317, WO2004058145, WO2003084930, WO2003084943, WO2004058744, WO2004013120, WO2004029204, WO2004035566, WO2004058249, WO2004058255, WO2004058727, WO2004069838, US20040214837, US20040214855, US20040214856, WO2004096209, WO2004096763, WO2004096794, WO2005000809, WO2004099157, US20040266845, WO2004110453, WO2004108728, WO2004000817, WO2005000820, US20050009870, WO200500974, WO2004111033-34, WO200411038-39, WO2005016286, WO2005007111, WO2005007628, US20050054679, WO2005027837, WO2005028456, WO2005063761-62, WO2005061509, WO2005077897, WO2006047516, WO2006060461, WO2006067428, WO2006067443, WO2006087480, WO2006087476, WO2006100208, WO2006106054, WO2006111849, WO2006113704, WO2007009705, WO2007017124, WO2007017126, WO2007018459, WO2007016460, WO2007020502, WO2007026215, WO2007028849, WO2007031720, WO2007031721, WO2007036945, WO2007038045, WO2007039740, US20070015810, WO2007046548, WO2007047737 beschrieben sind);
Cannabinoid Rezeptor 1 / Cannabinoid Rezeptor 2 (CB1/CB2) modulierende Verbindungen wie sie z.B. in WO2007001939, WO2007044215, WO2007047737 beschrieben sind; MC4-Agonisten (Melanocortin-4); z.B. 1-Amino-1,2,3,4-tetrahydro-naphthalin-2-carbonsäure [2-(3a-benzyl-2-methyl-3-oxo-2,3,3a,4,6,7-hexahydro-pyrazolo[4,3-c]pyridin-5-yl)-1-(4-chloro-phenyl)-2-oxo-ethyl]-amid; (WO 01/91752)) oder LB53280, LB53279, LB53278 oder THIQ, MB243, RY764, CHIR-785, PT-141 oder solche wie sie in WO2005060985, WO2005009950, WO2004087159, WO2004078717, WO2004078716, WO2004024720, US20050124652, WO2005051391, WO2004112793, WOUS20050222014, US20050176728, US20050164914, US20050124636, US20050130988, US20040167201, WO2004005324, WO2004037797, WO2005042516, WO2005040109, WO2005030797, US20040224901, WO200501921, WO200509184, WO2005000339, EP1460069, WO2005047253, WO2005047251, WO2005118573, EP1538159, WO2004072076, WO2004072077, WO2006021655-57, WO2007009894, WO2007015162, WO2007041061, WO2007041052 beschrieben sind;
Orexin-Rezeptor Antagonisten (z.B. 1-(2-Methyl-benzoxazol-6-yl)-3-[1,5]naphthyridin-4-yl-harnstoff Hydrochlorid (SB-334867-A) oder solche, wie sie z. B. in WO200196302, WO200185693, WO2004085403, WO2005075458, WO2006067224 beschrieben sind); Histamin H3 Rezeptor Agonisten (z. B. 3-Cyclohexyl-1-(4,4-dimethyl-1,4,6,7-tetrahydro-imidazo[4,5-c]pyridin-5-yl)-propan-1-on Oxalsäuresalz (WO 00/63208) oder solche, wie sie in WO200064884, WO2005082893, WO2006107661, WO2007003804, WO2007016496, WO2007020213 beschrieben sind);
Histamin H1 / Histamin H3 Modulatoren, wie z. B. Betahistin bzw. seinem Dihydrochlorid; CRF-Antagonisten (z.B. [2-Methyl-9-(2,4,6-trimethyl-phenyl)-9H-1,3,9-triaza-fluoren-4-yl]-dipropyl-amin (WO 00/66585));
CRF BP-Antagonisten (z.B. Urocortin);
Urocortin-Agonisten;
Agonisten des beta-3 Adrenoceptors wie z.B. 1-(4-Chloro-3-methanesulfonylmethyl-phenyl)-2-[2-(2,3-dimethyl-1H-indol-6-yloxy)-ethylamino]-ethanol Hydrochlorid (WO 01/83451) oder Solabegron (GW-427353) oder N-5984 (KRP-204) oder solche, wie sie in JP2006111553, WO2002038543, WO2007048840-843 beschrieben sind;
MSH (Melanocyt-stimulierendes Hormon)-Agonisten;
MCH (melanin-konzentrierendes Hormon) Rezeptor Antagonisten (wie z. B. NBI-845, A-761, A-665798, A-798, ATC-0175, T-226296, T-71, GW-803430 oder solche Verbindungen, wie sie in WO2005085200, WO2005019240, WO2004011438, WO2004012648, WO2003015769, WO2004072025, WO2005070898, WO2005070925, WO2004039780, WO2004092181, WO2003033476, WO2002006245, WO2002089729, WO2002002744, WO2003004027, FR2868780, WO2006010446, WO2006038680, WO2006044293, WO2006044174, JP2006176443, WO2006018280, WO2006018279, WO2006118320, WO2006130075, WO2007018248, WO2007012661, WO2007029847, WO2007024004, WO2007039462, WO2007042660, WO2007042668, WO2007042669, US2007093508, US2007093509, WO2007048802, JP2007091649 beschrieben sind);
CCK-A Agonisten (wie z.B. {2-[4-(4-Chloro-2,5-dimethoxy-phenyl)-5-(2-cyclohexyl-ethyl)-thiazol-2-ylcarbamoyl]-5,7-dimethyl-indol-1-yl}-essigsäure (WO 99/15525) oder SR-146131 (WO 0244150) oder SSR-125180) oder solchen, wie sie in WO2005116034 beschrieben sind; Serotonin-Wiederaufnahme-Inhibitoren (z.B. Dexfenfluramine);
gemischte Serotonin-/Dopamin-Wiederaufnahme-Inhibitoren (z.B. Bupropion) oder feste Kombinationen von Bupropion mit Naltrexon;
gemischte Sertonin- und noradrenerge Verbindungen (z.B. WO 00/71549);
5-HT-Rezeptor Agonisten z.B. 1-(3-Ethyl-benzofuran-7-yl)-piperazin Oxalsäuresalz (WO 01/09111);
gemischte Dopamin/Norepinephrin/Acetylcholin-Wiederaufnahme-Inhibitoren (z.B. Tesofensine);
5-HT2C Rezeptor Agonisten (wie z.B. Lorcaserin Hydrochlorid (APD-356) oder BVT-933 oder solche, wie sie in WO200077010, WO200077001-02, WO2005019180, WO2003064423, WO200242304, WO2005035533, WO2005082859, WO2006077025, WO2006103511 beschrieben sind);
5-HT6 Rezeptor Modulatoren, wie z.B. E-6837 oder BVT-74316 oder solche wie sie z.B. in WO2005058858, WO2007054257 beschrieben sind;
Bombesin-Rezeptor Agonisten (BRS-3 Agonisten;
Galanin-Rezeptor Antagonisten;
Wachstumshormon (z.B. humanes Wachstumshormon oder AOD-9604);
Wachstumshormon freisetzende Verbindungen (6-Benzyloxy-1-(2-diisopropylamino-ethylcarbamoyl)-3,4-dihydro-1H-isochinolin-2-carbonsäuretertiärbutylester (WO 01/85695)); Growth Hormone Secretagogue Receptor Antagonisten (Ghrelin Antagonisten) wie z. B. A-778193 oder solchen, wie sie in WO2005030734 beschrieben sind;
TRH-Agonisten (Thyrotrophin-releasing hormone, siehe z.B. EP 0 462 884);
entkoppelnde Protein 2- oder 3-Modulatoren;
Leptinagonisten (siehe z.B. Lee, Daniel W.; Leinung, Matthew C.; Rozhavskaya-Arena, Marina; Grasso, Patricia. Leptin agonists as a potential approach to the treatment of obesity. Drugs of the Future (2001), 26(9), 873-881);
DA-Agonisten (Dopamine-Agonisten; Bromocriptin, Doprexin);
Lipase/Amylase-Inhibitoren (z.B. WO 00/40569);
Inhibitoren der Diacylglycerol O-Acyltransferasen (DGATs) wie z. B. BAY-74-4113 oder wie z. B. in US2004/0224997, WO2004094618, WO200058491, WO2005044250,
WO2005072740, JP2005206492, WO2005013907, WO2006004200, WO2006019020, WO2006064189, WO2006082952, WO2006120125, WO2006113919, WO2006134317, WO2007016538 beschrieben;
Inhibitoren der Fettsäuresynthase (FAS) wie z.B. C75 oder solchen, wie in WO2004005277 beschrieben;
Inhibitoren der Stearoyl-CoA delta9 Desaturase (SCD1) wie sie z.B. in WO2007009236, WO2007044085, WO2007046867, WO2007046868, WO20070501124 beschrieben sind; Oxyntomodulin;
Oleoyl-Estron;
oder Agonisten oder partiellen Agonisten des Schilddrüsenhormonrezeptors (thyroid hormone receptor agonists) wie z. B: KB-2115 oder solche, wie in WO20058279, WO200172692, WO200194293, WO2003084915, WO2004018421, WO2005092316, WO2007003419, WO2007009913, WO2007039125 beschrieben, verabreicht.

Bei einer Ausführungsform ist der weitere Wirkstoff Varenicline Tartrate, ein partieller Agonist des alpha 4-beta 2 nikotinischen Acetylcholinrezeptors.

Bei einer Ausführungsform ist der weitere Wirkstoff Trodusquemine.

Bei einer Ausführungsform ist der weitere Wirkstoff ein Modulator des Enzyms SIRT1.

Bei einer Ausführungsform der Erfindung ist der weitere Wirkstoff Leptin;
siehe z.B. "Perspectives in the therapeutic use of leptin", Salvador, Javier; Gomez-Ambrosi, Javier; Fruhbeck, Gema, Expert Opinion on Pharmacotherapy (2001), 2(10), 1615-1622.

Bei einer Ausführungsform ist der weitere Wirkstoff Dexamphetamin oder Amphetamin.

Bei einer Ausführungsform ist der weitere Wirkstoff Fenfluramin oder Dexfenfluramin.

Bei noch einer Ausführungsform ist der weitere Wirkstoff Sibutramin.

Bei einer Ausführungsform ist der weitere Wirkstoff Mazindol oder Phentermin.

Bei einer Ausführungsform ist der weitere Wirkstoff ein Diphenylazetidinonderivat, wie z.B. in US 6,992,067 oder US 7,205,290 beschrieben.

Bei einer Ausführungsform wird die Verbindung der Formel I in Kombination mit Ballaststoffen, vorzugsweise unlöslichen Ballaststoffen (siehe z.B. Carob/ Caromax^{®} (Zunft H J; et al., Carob pulp preparation for treatment of hypercholesterolemia, ADVANCES IN THERAPY (2001 Sep-Oct), 18(5), 230-6) Caromax ist ein Carob enthaltendes Produkt der Fa. Nutrinova, Nutrition Specialties &Food Ingredients GmbH, Industriepark Höchst, 65926 Frankfurt / Main) verabreicht. Die Kombination mit Caromax^{®} kann in einer Zubereitung erfolgen, oder durch getrennte Gabe von Verbindungen der Formel I und Caromax^{®}. Caromax^{®} kann dabei auch in Form von Lebensmitteln, wie z.B. in Backwaren oder Müsliriegeln, verabreicht werden.

Es versteht sich, dass jede geeignete Kombination der erfindungsgemäßen Verbindungen mit einer oder mehreren der vorstehend genannten Verbindungen und wahlweise einer oder mehreren weiteren pharmakologisch wirksamen Substanzen als unter den Schutzbereich der vorliegenden Erfindung fallend angesehen wird.

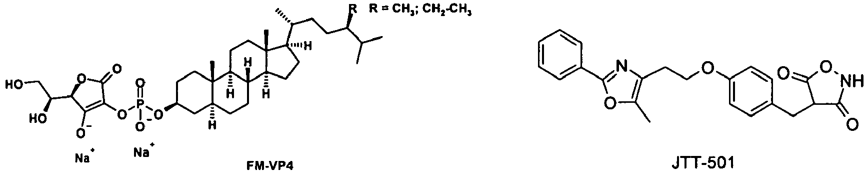

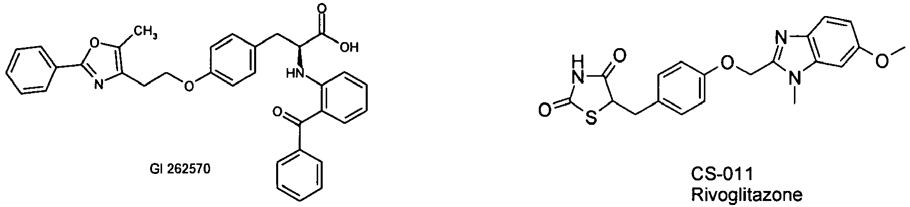

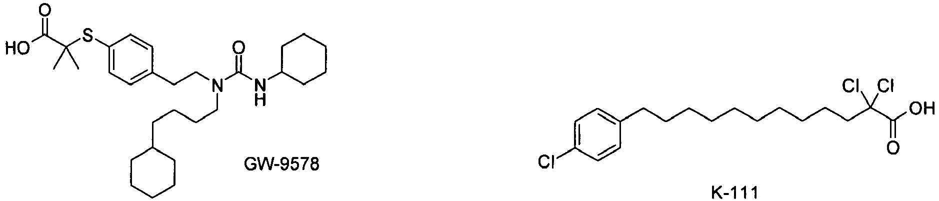

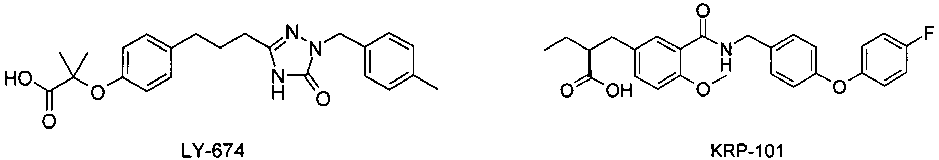

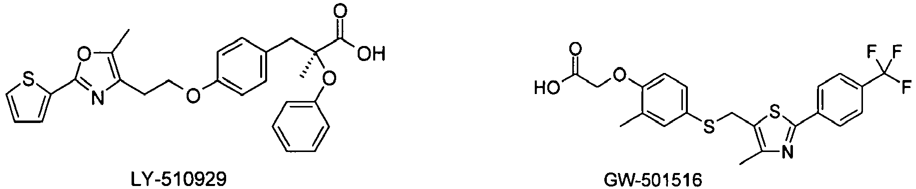

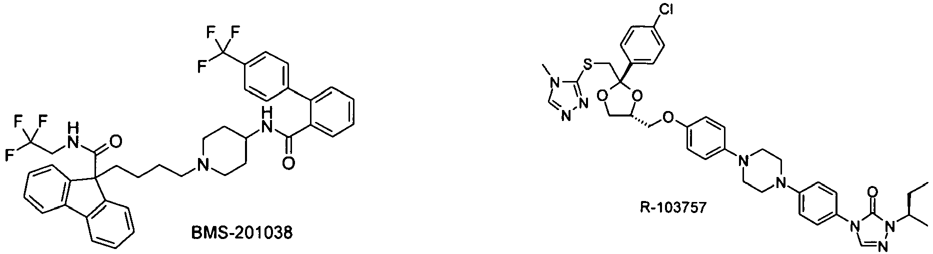

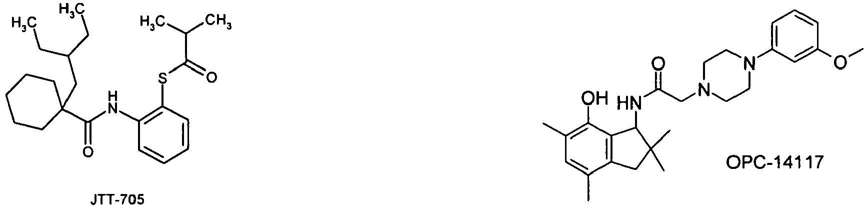

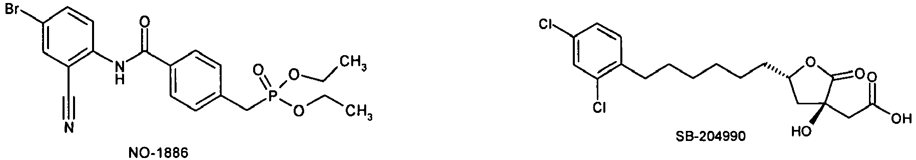

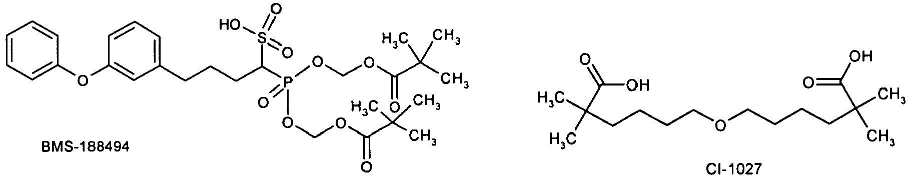

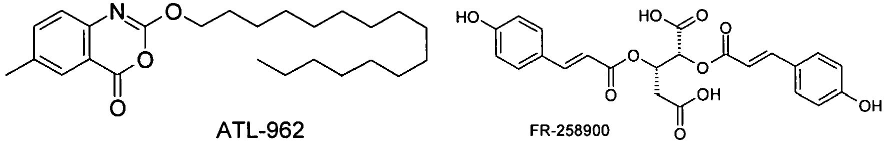

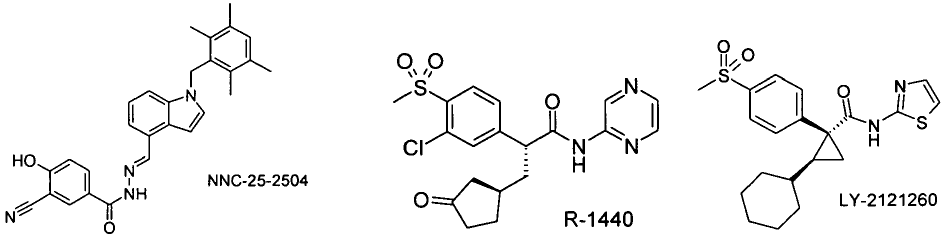

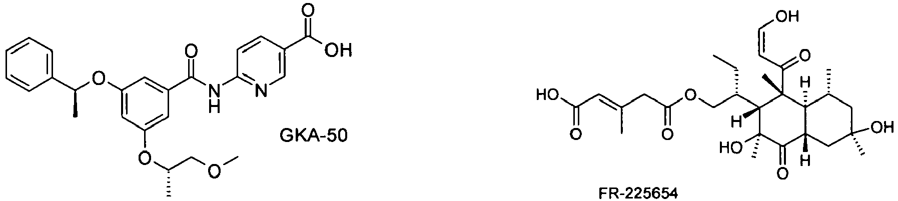

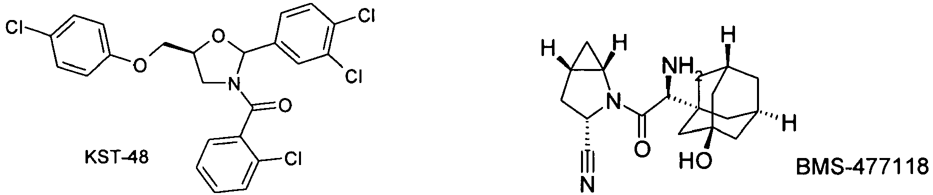

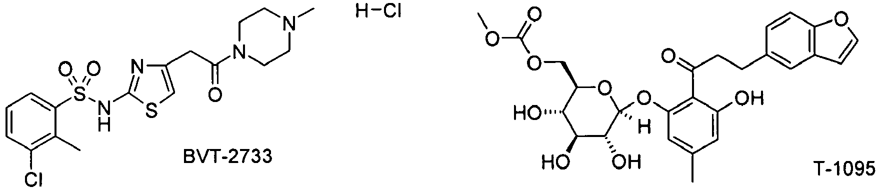

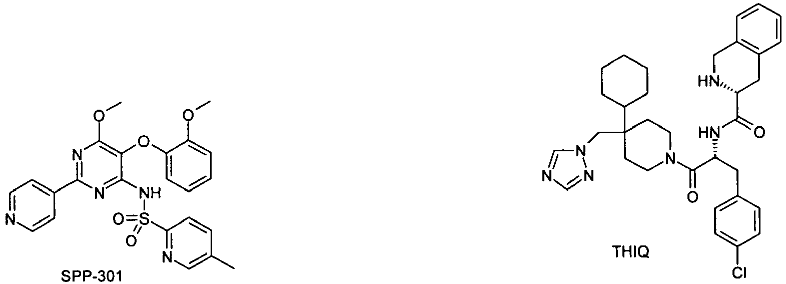

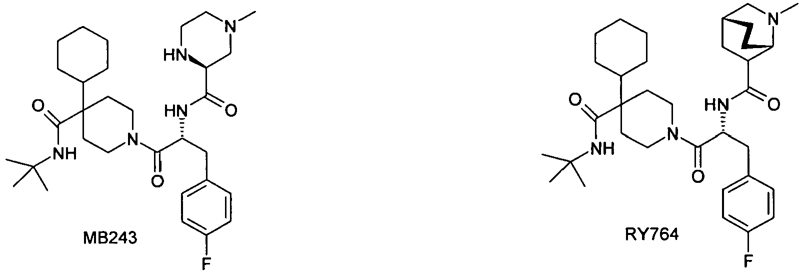

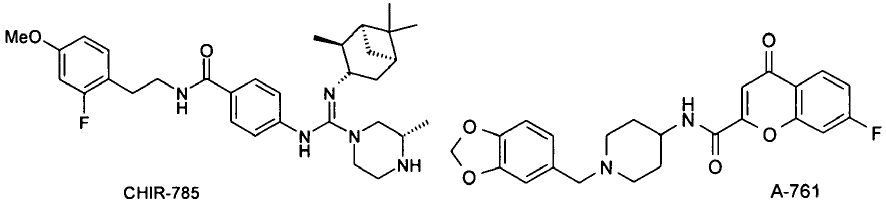

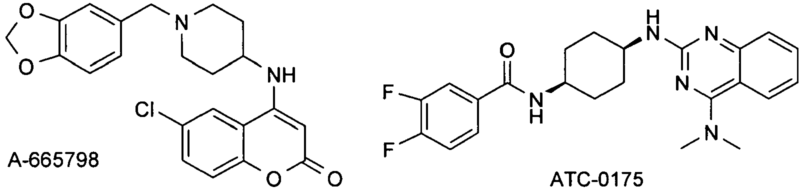

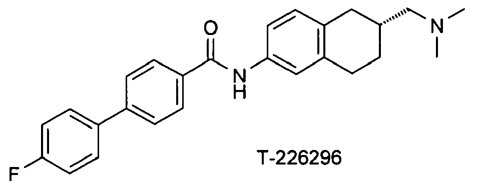

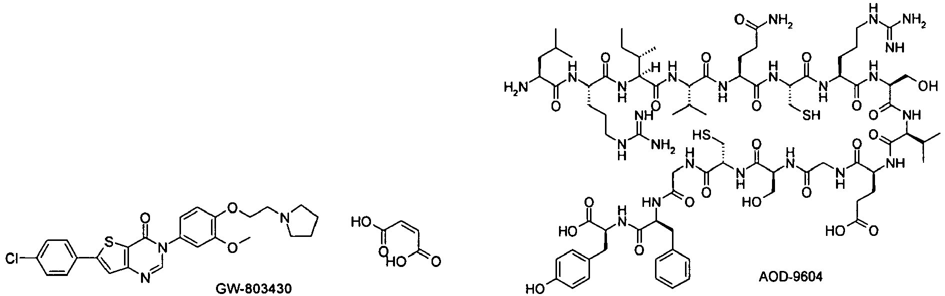

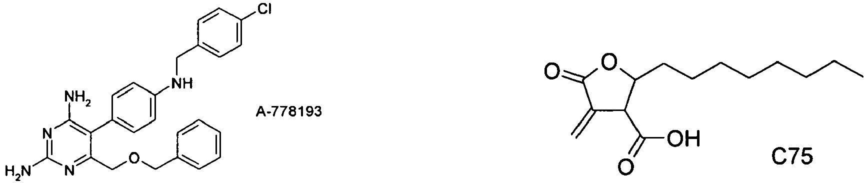

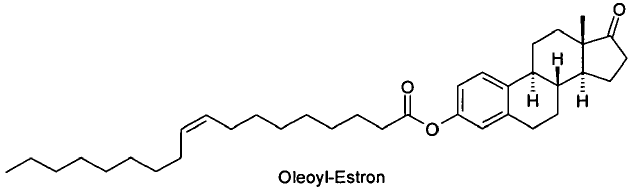

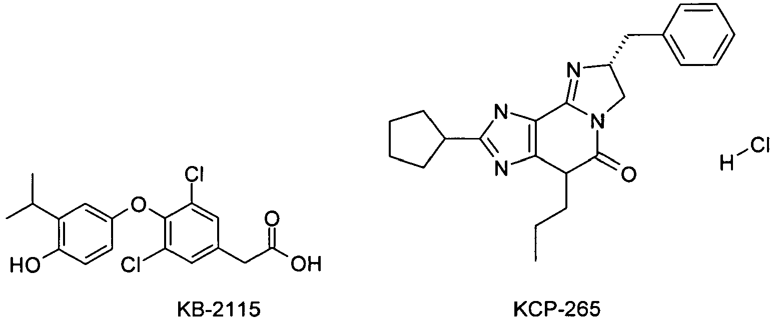

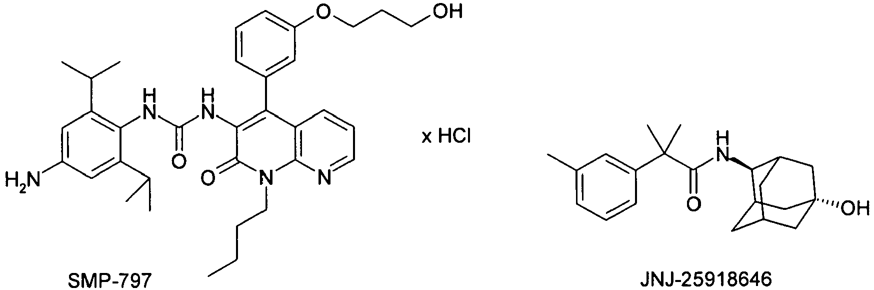

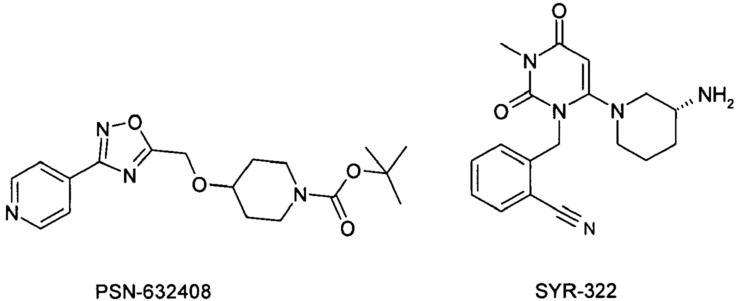

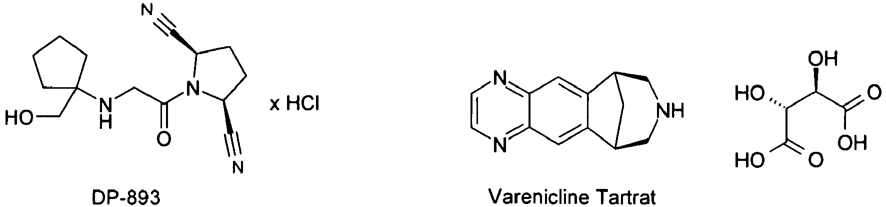

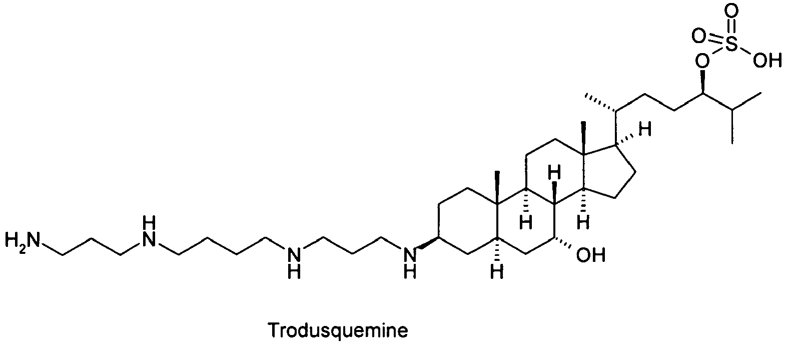

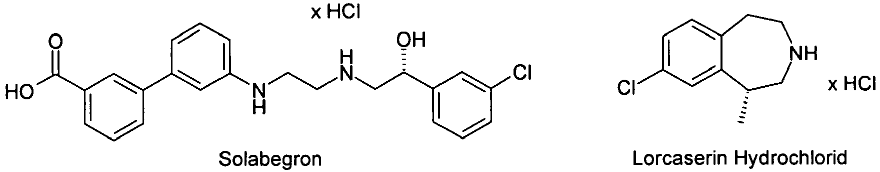

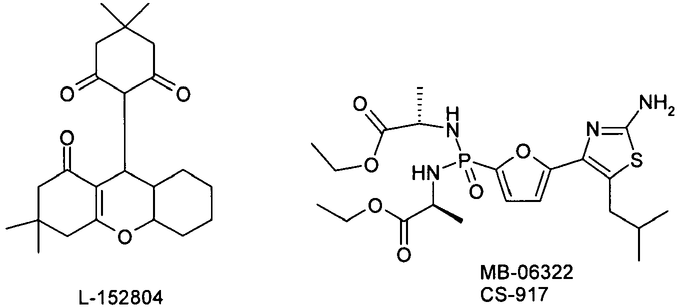

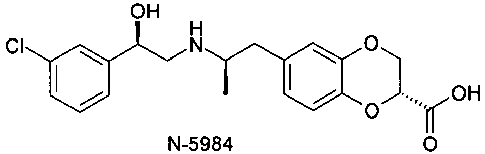

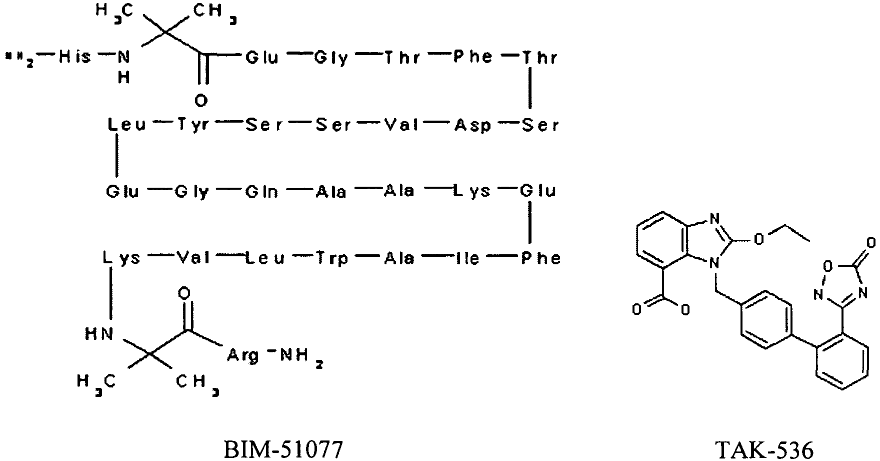

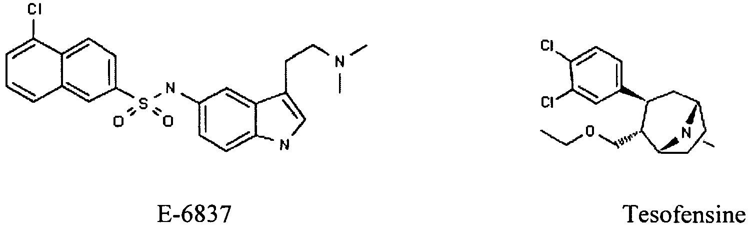

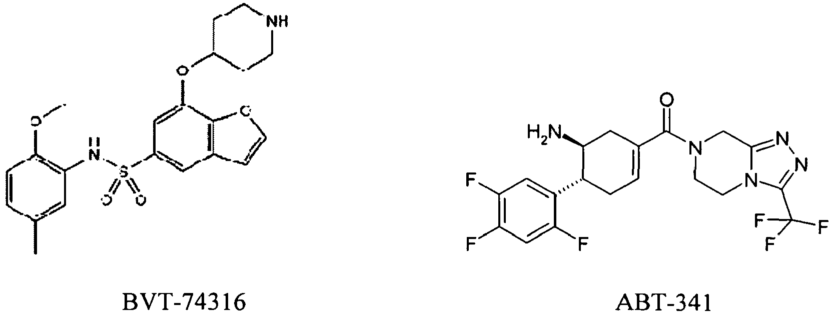

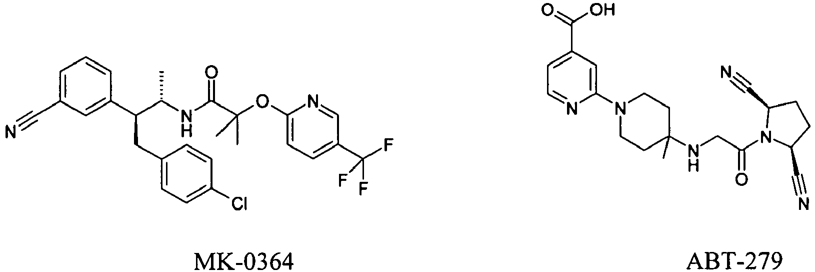

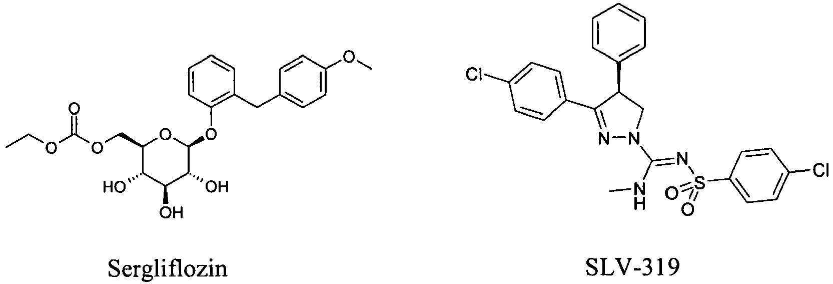

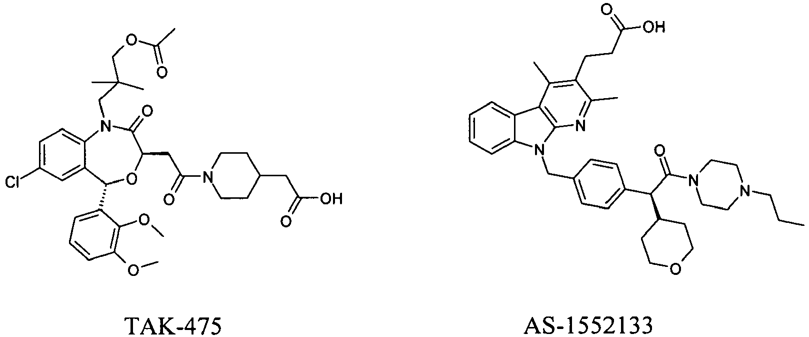

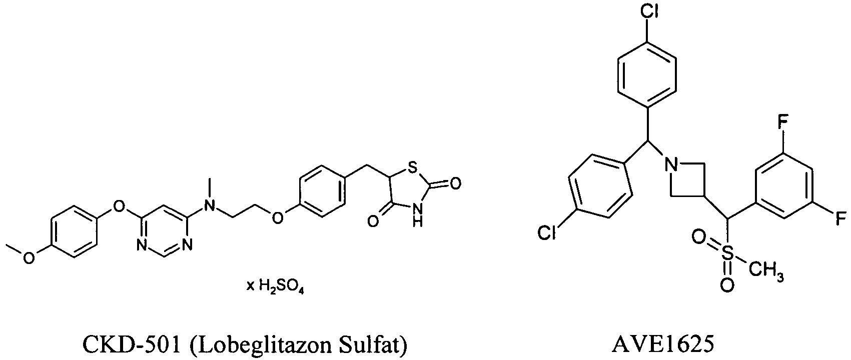

Die nachfolgend aufgeführten Beispiele dienen zur Erläuterung der Erfindung, ohne diese jedoch einzuschränken.

**Tabelle 1:**

| | | | | | | | | | | |
|---|---|---|---|---|---|---|---|---|---|---|
| 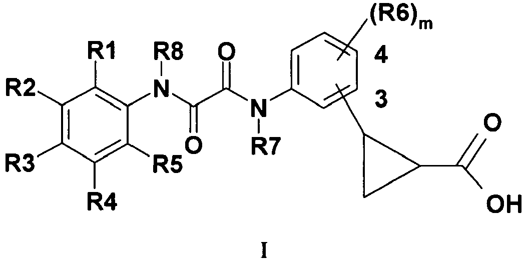 | | | | | | | | | | |

| **Bsp.** | **R1** | **R2** | **R3** | **R4** | **R5** | **R6** | **m** | **R7** | **R8** | **Verknüpfung** |
|---|---|---|---|---|---|---|---|---|---|---|
| 1 | Br | H | i-Pr | H | H | - | 0 | H | H | 4 |
| 2 | CF₃ | H | Cl | H | H | - | 0 | H | H | 4 |
| 3 | Cl | Cl | H | H | H | - | 0 | H | H | 4 |
| 4 | CF₃ | H | i-Pr | H | H | - | 0 | H | H | 4 |

Die Wirksamkeit der Verbindungen wurde wie folgt getestet:

### In-vitro FLIPR-Assay mit rekombinanten Zellen, die den GPCR GPR40 exprimieren

Funktionsüberprüfende Assays wurden mittels der FLIPR-Technik ("Fluorescence Imaging Plate Reader", Molecular Devices Corp.) durchgeführt. Hierzu wurden agonist-induzierte Änderungen der intrazellulären Konzentration von Ca²⁺ in rekombinanten HEK293 Zellen bestimmt, die den GPCR GPR40 exprimierten.

Für die Untersuchungen wurden Zellen in 96-well-Mikrotiterplatten (60000 Zellen/well) ausgesät und über Nacht wachsen gelassen. Das Medium wurde entfernt und die Zellen in Puffer inkubiert, der den Fluoreszenzfarbstoff Fluo-4 enthielt. Nach dieser Beladung mit Farbstoff wurden die Zellen gewaschen, Testsubstanz zugegeben und Änderungen in der intrazellulären Ca²⁺-Konzentration im FLIPR-Gerät gemessen. Ergebnisse wurden als prozentuale Änderung relativ zur Kontrolle dargestellt (0 %: keine Testsubstanz addiert; 100 %: 10 µM Referenzagonist Linolsäure addiert). zur Berechnung von Dosis/Wirkungskurven verwendet und EC₅₀-Werte bestimmt.

**Tabelle 2: Biologische Aktivität**

| **Bsp.** | **EC₅₀ [µM]** |
|---|---|
| 2 | 0,7 |
| 3 | 0,8 |

Aus der Tabelle ist abzulesen, dass die Verbindungen der Formel I den GPR40 Rezeptor aktivieren und dadurch gut zur Behandlung von Hyperglykämie und von Diabetes geeignet sind. Durch die Verbindungen der Formel I wird die Insulinausschüttung erhöht (siehe Itoh et al., Nature 2003, 422, 173-176).

Aufgrund der Aktivierung des GPR40 Rezeptors können die Verbindungen der Formel I auch zur Behandlung bzw. Prävention weiterer Krankheiten angewendet werden.
Die Verbindungen der vorliegenden Erfindung eignen sich insbesondere zur Behandlung und/oder Prävention von:
1.
   - Störungen des Fettsäurestoffwechsels und Glucoseverwertungsstörungen
   - Störungen, bei denen Insulinresistenz eine Rolle spielt
2. Diabetes mellitus, insbesondere Typ-2-Diabetes, einschließlich der Prävention der damit verbundenen Folgeerkrankungen.
   - Besondere Aspekte in diesem Zusammenhang sind
   - Hyperglykämia,
   - Verbesserung der Insulinresistenz,
   - Verbesserung der Glucosetoleranz,
   - Schutz der ß-Zellen der Bauchspeicheldrüse
   - Prävention makro- und mikrovaskulärer Erkrankungen
3. Verschiedene andere leiden, die mit dem metabolischen Syndrom bzw. Syndrom X assoziert sein können, wie
   - Zunahme des Bauchumfangs
   - Dyslipidämie (z.B. Hypertriglyceridämie und/oder niedriges HDL)
   - Insulinresistenz
   - Hyperkoagulabilität
   - Hyperurikämie
   - Mikroalbuminämie
   - Thrombosen, hyperkoagulabile und prothrombotische Zustände (arteriell und venös)
   - Bluthochdruck
   - Herzinsuffizienz, beispielsweise (jedoch nicht darauf beschränkt), nach Herzinfarkt, hypertensiver Herzkrankheit oder Kardiomyopathie
4. Gedächtnisstörungen, geistige Defekte, ZNS-Erkrankungen wie
   - Altersdemenzen
   - Alzheimer'schen Krankheit
   - Behandlung verminderter Aufmerksamkeit oder Wachsamkeit
   - Schizophrenie

Die Verbindungen der Formel I lassen sich z. B. dadurch herstellen, dass man geeignete Ausgangsstoffe der Formel II 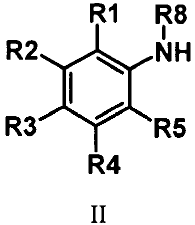 mit Oxalylchlorid in die Oxalamid-säurechloride der Formel III 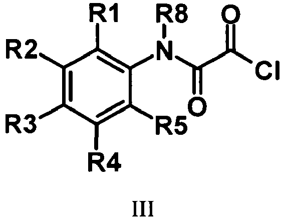 in geeigenten Lösungsmitteln, wie zum Beispiel Essigsäureethylester oder Acetonitril, umsetzt. Die so hergestellten Verbindungen der Formel III werden mit Aminophenyl-substituierten Carbonsäuren der Formel IV 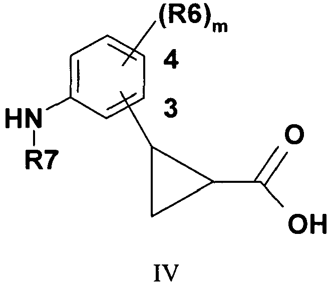 in geeigneten Lösungsmittel wie zum Beispiel Acetonitril, 1,2-Dichlorethan oder Dichlormethan bei geeigneten Temperaturen, vorzugsweise in der Siedehitze, zu Verbindungen der Formel I umgesetzt.

Nachfolgend wird die allgemeine Herstellung der Beispiele detailliert beschrieben:
Experimenteller Teil:
   Allgemeine Versuchsvorschrift:
      Herstellung des Oxalamid-säurechlorides:
         50 mg eines Anilins werden in 3 ml Dichlorethan gelöst und mit 38 µl Oxalylchlorid versetzt. Die Reaktionslösung wird 16 Stunden bei 100°C gerührt und anschließend eingeengt.
   Herstellung der Oxalsäurebisamide der Formel I:
      Die Aminophenyl-substituierte Cyclopropylcarbonsäure wird zu einer Suspension des Oxalamidsäurechlorids in 3 ml Acetonitril gegeben und 15 Stunden bei 90°C gerührt. Der entstandenen Niederschlag wird abgesaugt und falls nötig mittel Flashchromatographie (SiO₂, Dichlormethan-Isopropanol) gereinigt.
      Die Analyse der Verbindungen erfolgte mittels LC/MS. Der entsprechende Molpeak (M+H)⁺ konnte bei allen Beispielen per LC/MS detektiert werden.

## Patentansprüche

1. Verbindungen der Formel I, 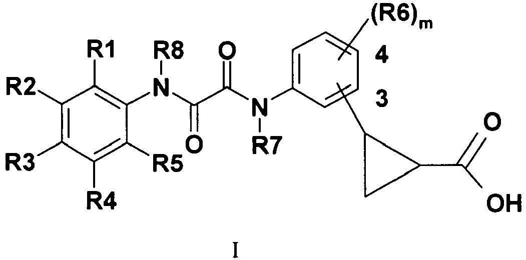 worin bedeuten
R1, R2, R3, R4, R5 unabhängig voneinander H, F, Cl, Br, CN, CF₃, OH, OCF₃, OCHF₂, SCH₃, SCF₃, Phenyl, OPhenyl, COOH, COO-(C₁-C₆)-Alkyl, CO-(C₁-C₆)-Alkyl, (C₁-C₆)-Alkyl, O-(C₁-C₆)-Alkyl, OBn, SO₂-(C₁-C₄)-Alkyl, SO₃H, SO₂NR9R10, NR9R10 oder SO₂-N-Piperidinyl, wobei Alkyl und Phenyl ein oder mehrfach mit R12 substituiert sein können;
R7, R8 unabhängig voneinander H oder (C₁-C₆)-Alkyl;
m 0, 1, 2, 3 oder 4;
R6 OH, F, Cl, Br, CN, OCH₃, OCF₃, CH₃, CF₃, (C₁-C₆)-Alkyl oder O-(C₁-C₆)-Alkyl, wobei Alkyl ein oder mehrfach mit OH, F, Cl, Br oder CN substituiert sein kann;
R9, R10 unabhängig voneinander H, (C₁-C₆)-Alkyl oder Phenyl, wobei Alkyl ein oder mehrfach substituiert sein kann mit F, Cl oder Br, und Phenyl ein oder mehrfach mit R6 substituiert sein kann;
R11 F, Cl, Br, CN, OH, O-(C₁-C₆)-Alkyl, (C₁-C₆)-Alkyl, (C₂-C₆)-Alkenyl, (C₂-C₆)-Alkinyl oder NR9R10;
R12 F, Cl, Br, CN, OH, O-(C₁-C₆)-Alkyl, (C₁-C₆)-Alkyl, (C₂-C₆)-Alkenyl, (C₂-C₆)-Alkinyl, NR9R10, COOH, COO-(C₁-C₄)-Alkyl, SCH₃, SCF₃, SO₂-(C₁-C₄)-Alkyl, SO₃H oder SO₂NR9R10;
und deren physiologisch verträgliche Salze.

2. Verbindungen der Formel I, gemäß Anspruch 1, **dadurch gekennzeichnet, dass** darin bedeuten
R1, R2, R3, R4, R5 unabhängig voneinander H, F, Cl, Br, CN, CF₃, OH, OCF₃, OCHF₂, SCH₃, SCF₃, Phenyl, OPhenyl, COOH, COO-(C₁-C₆)-Alkyl, CO-(C₁-C₆)-Alkyl, (C₁-C₆)-Alkyl, O-(C₁-C₆)-Alkyl, OBn, SO₂-(C₁-C₄)-Alkyl, SO₃H, SO₂NR9R10, NR9R10 oder SO₂-N-Piperidinyl, wobei Alkyl und Phenyl ein oder mehrfach mit R12 substituiert sein können;
R7, R8 H;
m 0, 1, 2, 3 oder 4;
R6 OH, F, Cl, Br, CN, OCH₃, OCF₃, CH₃, CF₃, (C₁-C₆)-Alkyl oder O-(C₁-C₆)-Alkyl, wobei Alkyl ein oder mehrfach mit OH, F, Cl, Br oder CN substituiert sein kann;
R9, R10 unabhängig voneinander H, (C₁-C₆)-Alkyl oder Phenyl, wobei Alkyl ein oder mehrfach substituiert sein kann mit F, Cl oder Br, und Phenyl ein oder mehrfach mit R6 substituiert sein kann;
R11 F, Cl, Br, CN, OH, O-(C₁-C₆)-Alkyl, (C₁-C₆)-Alkyl, (C₂-C₆)-Alkenyl, (C₂-C₆)-Alkinyl oder NR9R10;
R12 F, Cl, Br, CN, OH, O-(C₁-C₆)-Alkyl, (C₁-C₆)-Alkyl, (C₂-C₆)-Alkenyl, (C₂-C₆)-Alkinyl, NR9R10, COOH, COO-(C₁-C₄)-Alkyl, SCH₃, SCF₃, SO₂-(C₁-C₄)-Alkyl, SO₃H oder SO₂NR9R10;
und deren physiologisch verträgliche Salze.

3. Verbindungen gemäß Anspruch 1 oder 2, **dadurch gekennzeichnet, dass** darin bedeuten
R1 H, F, Cl, Br, CN, CF₃, OH, OCF₃, OCHF₂, SCH₃, SCF₃, Phenyl, OPhenyl, COOH, COO-(C₁-C₆)-Alkyl, CO-(C₁-C₆)-Alkyl, (C₁-C₆)-Alkyl, O-(C₁-C₆)-Alkyl, OBn;
R2 H, F, Cl, Br, CN, CF₃, OCF₃, OCHF₂, SCH₃, SCF₃, Phenyl, OPhenyl, COOH, COO-(C₁-C₆)-Alkyl, CO-(C₁-C₆)-Alkyl, (C₁-C₆)-Alkyl, O-(C₁-C₆)-Alkyl, OBn;
R3 H, F, Cl, Br, CN, CF₃, OH, OCF₃, OCHF₂, SCH₃, SCF₃, Phenyl, OPhenyl, COOH, COO-(C₁-C₆)-Alkyl, CO-(C₁-C₆)-Alkyl, (C₁-C₆)-Alkyl, O-(C₁-C₆)-Alkyl, OBn;
R4 H, F, Cl, Br, CN, CF₃, OCF₃, OCHF₂, SCH₃, SCF₃, Phenyl, OPhenyl, COOH, COO-(C₁-C₆)-Alkyl, CO-(C₁-C₆)-Alkyl, (C₁-C₆)-Alkyl, O-(C₁-C₆)-Alkyl, OBn;
R5 H, F, Cl, Br, CN, CF₃, OH, OCF₃, OCHF₂, SCH₃, SCF₃, Phenyl, OPhenyl, COOH, COO-(C₁-C₆)-Alkyl, CO-(C₁-C₆)-Alkyl, (C₁-C₆)-Alkyl, O-(C₁-C₆)-Alkyl, OBn;
wobei mindestens einer der Reste R1 bis R5 eine andere Bedeutung als Wasserstoff hat;
R7, R8 unabhängig voneinander H oder (C₁-C₆)-Alkyl;
m 0;
R9, R10 unabhängig voneinander H oder (C₁-C₆)-Alkyl, wobei Alkyl ein oder mehrfach substituiert sein kann mit F, Cl oder Br;
sowie deren physiologisch verträglichen Salze.

4. Verbindungen gemäß einem oder mehreren der Ansprüche 1 bis 3, zur Anwendung als Arzneimittel.

5. Arzneimittel enthaltend eine oder mehrere der Verbindungen gemäß einem oder mehreren der Ansprüche 1 bis 3.

6. Arzneimittel enthaltend eine oder mehrere der Verbindungen gemäß Anspruch 5 und mindestens einen weiteren Wirkstoff.

7. Arzneimittel, gemäß Anspruch 6, **dadurch gekennzeichnet, dass** es als weiteren Wirkstoff eine oder mehrere Antidiabetika, hypoglykämische Wirkstoffe, HMGCoA-Reduktase Inhibitoren, Cholesterinresorptionsinhibitoren, PPAR gamma Agonisten, PPAR alpha Agonisten, PPAR alpha/gamma Agonisten, PPAR delta Agonisten, Fibrate, MTP-Inhibitoren, Gallensäureresorptionsinhibitoren, CETP-Inhibitoren, polymere Gallensäureadsorber, LDL-Rezeptorinducer, ACAT-Inhibitoren, Antioxidantien, ATP-Citrat-Lyase Inhibitoren, Squalen Synthetase Inhibitoren, Lipoprotein(a) Antagonisten, HM74A Rezeptor Agonisten, Lipase Inhibitoren, Insuline, Sulfonylharnstoffe, Biguanide, Meglitinide, Thiazolidindione, α-Glukosidase-Inhibitoren, auf den ATP-abhängigen Kaliumkanal der Betazellen wirkende Wirkstoffe, Glykogen Phosphorylase Inhibitoren, Glukagon-Rezeptor-Antagonisten, Aktivatoren der Glukokinase, Inhibitoren der Glukoneogenese, Inhibitoren der Fructose-1,6-biphosphatase, Modulatoren des Glukosetransporters-4, Inhibitoren der Glutamin-Fructose-6-Phosphat-Amidotransferase, Inhibitoren der Dipeptidylpeptidase-IV, Hemmstoffe der 11-beta-Hydroxysteroid-Dehydrogenase-1, Inhibitoren der Protein-Tyrosin-Phosphatase-1B, Modulatoren des natrium-abhängigen Glukosetransporters 1 oder 2, Inhibitoren der hormon-sensitiven Lipase, Hemmstoffe der Acetyl-CoA Carboxylase, Inhibitoren der Phosphoenolpyruvatcarboxykinase, Inhibitoren der Glykogen Synthase Kinase-3 beta, Inhibitoren der Protein Kinase C beta, Endothelin-A-Rezeptor Antagonisten, Inhibitoren der I kappaB Kinase, Modulatoren des Glukocorticoidrezeptors, MC4-Agonisten, H3-Agonisten, TNF-Agonisten, CRF BP-Antagonisten, Urocortin-Agonisten, CB1-Rezeptor Antagonisten, MSH (Melanocyt-stimulierendes Hormon)-Agonisten, Serotonin-Wiederaufnahme-Inhibitoren, gemischte Serotonin- und noradrenerge Verbindungen, 5HT-Agonisten, Bombesin-Agonisten, Galanin-Antagonisten, Wachstumshormone, Wachstumshormon freisetzende Verbindungen, TRH-Agonisten, entkoppelnde Protein 2- oder 3-Modulatoren, Leptinagonisten, DA-Agonisten, Lipase/Amylase-Inhibitoren, PPAR-Modulatoren, RXR-Modulatoren oder TR-β-Agonisten oder Amphetamine enthält.

8. Verwendung der Verbindungen gemäß einem oder mehreren der Ansprüche 1 bis 3 zur Herstellung eines Medikamentes zur Blutzuckersenkung.

9. Verwendung der Verbindungen gemäß einem oder mehreren der Ansprüche 1 bis 3 zur Herstellung eines Medikamentes zur Behandlung des Diabetes.

10. Verwendung der gemäß einem oder mehreren der Ansprüche 1 bis 3 zur Herstellung eines Medikamentes zur Erhöhung der Insulinausschüttung.

11. Verfahren zur Herstellung eines Arzneimittels enthaltend eine oder mehrere der Verbindungen gemäß einem oder mehreren der Ansprüche 1 bis 3, **dadurch gekennzeichnet, dass** der Wirkstoff mit einem pharmazeutisch geeigneten Träger vermischt wird und diese Mischung in eine für die Verabreichung geeignete Form gebracht wird.

12. Verwendung der Verbindungen gemäß einem oder mehreren der Ansprüche 1 bis 3 zur Herstellung eines Medikamentes zur Behandlung von ZNS-Erkrankungen.

13. Verwendung der Verbindungen gemäß einem oder mehreren der Ansprüche 1 bis 3 zur Herstellung eines Medikamentes zur Behandlung von Schizophrenie.

14. Verwendung der Verbindungen gemäß einem oder mehreren der Ansprüche 1 bis 3 zur Herstellung eines Medikamentes zur Behandlung von Alzheimer.

## Claims

1. A compound of the formula I, 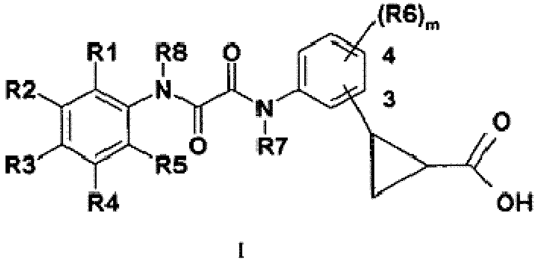 where
R1, R2, R3, R4, R5 are each independently H, F, Cl, Br, CN, CF₃, OH, OCF₃, OCHF₂, SCH₃, SCF₃, phenyl, Ophenyl, COOH, COO-(C₁-C₆)-alkyl, CO-(C₁-C₆)-alkyl, (C₁-C₆)-alkyl, O-(C₁-C₆)-alkyl, OBn, SO₂-(C₁-C₄)-alkyl, SO₃H, SO₂NR9R10, NR9R10 or SO₂-N-piperidinyl, wherein alkyl and phenyl may be substituted once or more than once by R12;
R7, R8 are each independently H or (C₁-C₆)-alkyl;
m is 0, 1, 2, 3 or 4;
R6 is OH, F, Cl, Br, CN, OCH₃, OCF₃, CH₃, CF₃, (C₁-C₆)-alkyl or O-(C₁-C₆)-alkyl, where alkyl may be substituted once or more than once by OH, F, Cl, Br or CN;
R9, R10 are each independently H, (C₁-C₆)-alkyl or phenyl, where alkyl may be substituted once or more than once by F, Cl or Br and phenyl may be substituted once or more than once by R6;
R11 is F, Cl, Br, CN, OH, O-(C₁-C₆)-alkyl, (C₁-C₆)-alkyl, (C₂-C₆)-alkenyl, (C₂-C₆)-alkynyl or NR9R10;
R12 is F, Cl, Br, CN, OH, O-(C₁-C₆)-alkyl, (C₁-C₆)-alkyl, (C₂-C₆)-alkenyl, (C₂-C₆)-alkynyl, NR9R10, COOH, COO-(C₁-C₄)-alkyl, SCH₃, SCF₃, SO₂-(C₁-C₄)-alkyl, SO₃H or SO₂NR9R10;
and physiologically compatible salts thereof.

2. A compound of the formula I, as claimed in claim 1, wherein
R1, R2, R3, R4, R5 are each independently H, F, Cl, Br, CN, CF₃, OH, OCF₃, OCHF₂, SCH₃, SCF₃, phenyl, Ophenyl, COOH, COO-(C₁-C₆)-alkyl, CO-(C₁-C₆)-alkyl, (C₁-C₆)-alkyl, O-(C₁-C₆)-alkyl, OBn, SO₂-(C₁-C₄)-alkyl, SO₃H, SO₂NR9R10, NR9R10 or SO₂-N-piperidinyl, wherein alkyl and phenyl may be substituted once or more than once by R12;
R7, R8 are H;
m is 0, 1, 2, 3 or 4;
R6 is OH, F, Cl, Br, CN, OCH₃, OCF₃, CH₃, CF₃, (C₁-C₆)-alkyl or O-(C₁-C₆)-alkyl, where alkyl may be substituted once or more than once by OH, F, Cl, Br or CN;
R9, R10 are each independently H, (C₁-C₆)-alkyl or phenyl, where alkyl may be substituted once or more than once by F, Cl or Br and phenyl may be substituted once or more than once by R6;
R11 is F, Cl, Br, CN, OH, O-(C₁-C₆)-alkyl, (C₁-C₆)-alkyl, (C₂-C₆)-alkenyl, (C₂-C₆)-alkynyl or NR9R10;
R12 is F, Cl, Br, CN, OH, O-(C₁-C₆)-alkyl, (C₁-C₆)-alkyl, (C₂-C₆)-alkenyl, (C₂-C₆)-alkynyl, NR9R10, COOH, COO-(C₁-C₄)-alkyl, SCH₃, SCF₃, SO₂-(C₁-C₄)-alkyl, SO₃H or SO₂NR9R10;
and physiologically compatible salts thereof.

3. A compound as claimed in claim 1 or 2, where
R1 is H, F, Cl, Br, CN, CF₃, OH, OCF₃, OCHF₂, SCH₃, SCF₃, phenyl, Ophenyl, COOH, COO-(C₁-C₆)-alkyl, CO-(C₁-C₆)-alkyl, (C₁-C₆)-alkyl, O-(C₁-C₆)-alkyl, OBn;
R2 is H, F, Cl, Br, CN, CF₃, OCF₃, OCHF₂, SCH₃, SCF₃, phenyl, Ophenyl, COOH, COO-(C₁-C₆)-alkyl, CO-(C₁-C₆)-alkyl, (C₁-C₆)-alkyl, O-(C₁-C₆)-alkyl, OBn;
R3 is H, F, Cl, Br, CN, CF₃, OH, OCF₃, OCHF₂, SCH₃, SCF₃, phenyl, Ophenyl, COOH, COO-(C₁-C₆)-alkyl, CO-(C₁-C₆)-alkyl, (C₁-C₆)-alkyl, O-(C₁-C₆)-alkyl, OBn;
R4 is H, F, Cl, Br, CN, CF₃, OCF₃, OCHF₂, SCH₃, SCF₃, phenyl, Ophenyl, COOH, COO- (C₁-C₆)-alkyl, CO-(C₁-C₆)-alkyl, (C₁-C₆)-alkyl, O-(C₁-C₆)-alkyl, OBn;
R5 is H, F, Cl, Br, CN, CF₃, OH, OCF₃, OCHF₂, SCH₃, SCF₃, phenyl, Ophenyl, COOH, COO-(C₁-C₆)-alkyl, CO-(C₁-C₆)-alkyl, (C₁-C₆)-alkyl, O-(C₁-C₆)-alkyl, OBn;
wherein at least one of the residues R1 to R5 is defined as other than hydrogen;
R7, R8 are each independently H or (C₁-C₆)-alkyl;
m is 0;
R9, R10 are each independently H or (C₁-C₆)-alkyl where alkyl may be substituted once or more than once by F, Cl or Br;
and also the physiologically compatible salts thereof.

4. The compound as claimed in one or more of claims 1 to 3 for use as a medicament.

5. A medicament comprising one or more compounds as claimed in one or more of claims 1 to 3.

6. The medicament comprising one or more compounds as claimed in claim 5 and at least one further active ingredient.

7. The medicament as claimed in claim 6, wherein said medicament comprises as further active ingredient one or more antidiabetics, hypoglycemic active ingredients, HMGCoA reductase inhibitors, cholesterol absorption inhibitors, PPAR gamma agonists, PPAR alpha agonists, PPAR alpha/gamma agonists, PPAR delta agonists, fibrates, MTP inhibitors, bile acid absorption inhibitors, CETP inhibitors, polymeric bile acid adsorbers, LDL receptor inducers, ACAT inhibitors, antioxidants, ATP citrate lyase inhibitors, squalene synthetase inhibitors, lipoprotein(a) antagonists, HM74A receptor agonists, lipase inhibitors, insulins, sulfonylureas, biguanides, meglitinides, thiazolidinediones, α-glucosidase inhibitors, active ingredients acting on the ATP-dependent potassium channel of beta cells, glycogen phosphorylase inhibitors, glucagon receptor antagonists, activators of glucokinase, inhibitors of gluconeogenesis, inhibitors of fructose-1,6-biphosphatase, modulators of glucose transporter type 4, inhibitors of the glutamine-fructose-6-phosphate amidotransferase, inhibitors of dipeptidylpeptidase IV, inhibitors of 11-beta-hydroxysteroid dehydrogenase type 1, inhibitors of protein tyrosine phosphatase 1 B, modulators of the sodium-dependent glucose transporters 1 or 2, inhibitors of hormone-sensitive lipase, inhibitors of acetyl-CoA carboxylase, inhibitors of phosphoenolpyruvate carboxykinase, inhibitors of glycogen synthase kinase-3 beta, inhibitors of protein kinase C beta, endothelin-A receptor antagonists, inhibitors of I kappa B kinase, modulators of the glucocorticoid receptor, MC4 agonists, H3 agonists, TNF agonists, CRF BP antagonists, urocortin agonists, CB1 receptor antagonists, MSH (melanocyte-stimulating hormone) agonists, serotonin reuptake inhibitors, mixed serotonin and noradrenergic compounds, 5HT agonists, bombesin agonists, galanin antagonists, growth hormones, compounds releasing growth hormone TRH agonists, decoupling protein-2 or -3 modulators, leptin agonists, DA agonists, lipase/amylase inhibitors, PPAR modulators, RXR modulators or TR-β-agonists or amphetamines.

8. The use of the compounds as claimed in one or more of claims 1 to 3 for preparing a medicament for lowering blood sugar.

9. The use of the compounds as claimed in one or more of claims 1 to 3 for preparing a medicament for the treatment of diabetes.

10. The use of as claimed in one or more of claims 1 to 3 for preparing a medicament for increasing insulin secretion.

11. A method for preparing a medicament comprising one or more of the compounds as claimed in one or more of claims 1 to 3, wherein the active ingredient is mixed with a pharmaceutically suitable carrier and this mixture is converted into a form suitable for administration.

12. The use of the compounds as claimed in one or more of claims 1 to 3 for preparing a medicament for the treatment of CNS disorders.

13. The use of the compounds as claimed in one or more of claims 1 to 3 for preparing a medicament for the treatment of schizophrenia.

14. The use of the compounds as claimed in one or more of claims 1 to 3 for preparing a medicament for the treatment of Alzheimers.

## Revendications

1. Composés de formule I, 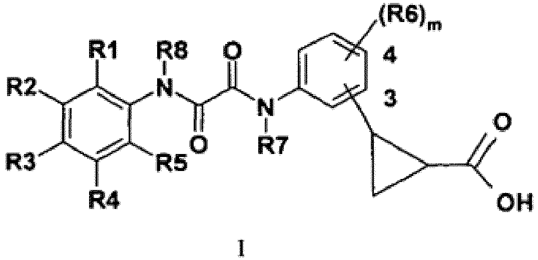 dans laquelle
R1, R2, R3, R4, R5 signifient, indépendamment les uns des autres, H, F, CI, Br, CN, CF₃, OH, OCF₃, OCHF₂, SCH₃, SCF₃, phényle, O-phényle, COOH, COO-(C₁-C₆)-alkyle, CO-(C₁-C₆)-alkyle, (C₁-C₆)-alkyle, O-(C₁-C₆)-alkyle, OBn, SO₂-(C₁-C₄)-alkyle, SO₃H, SO₂NR9R10, NR9R10 ou SO₂-N-pipéridinyle, alkyle et phényle pouvant être monosubstitués ou polysubstitués par R12 ;
R7, R8 signifient, indépendamment l'un de l'autre, H ou (C₁-C₆)-alkyle ;
m vaut 0, 1, 2, 3 ou 4 ;
R6 signifie OH, F, CI, Br, CN, OCH₃, OCF₃, CH₃, CF₃, (C₁-C₆)-alkyle ou O-(C₁-C₆)-alkyle, alkyle pouvant être monosubstitué ou polysubstitué par OH, F, CI, Br ou CN ;
R9, R10 signifient, indépendamment l'un de l'autre, H, (C₁-C₆)-alkyle ou phényle, alkyle pouvant être monosubstitué ou polysubstitué par F, Cl ou Br et phényle pouvant être monosubstitué ou polysubstitué par R6 ;
R11 signifie F, CI, Br, CN, OH, O-(C₁-C₆)-alkyle, (C₁-C₆)-alkyle, (C₂-C₆)-alcényle, (C₂-C₆)-alcynyle ou NR9R10 ;
R12 signifie F, CI, Br, CN, OH, O-(C₁-C₆)-alkyle, (C₁-C₆)-alkyle, (C₂-C₆)-alcényle, (C₂-C₆)-alcynyle, NR9R10, COOH, COO-(C₁-C₄)-alkyle, SCH₃, SCF₃, SO₂-(C₁-C₄)-alkyle, SO₃H ou SO₂NR9R10 ;
et leurs sels physiologiquement acceptables.

2. Composés de formule I, selon la revendication 1, **caractérisés en ce que**
R1, R2, R3, R4, R5 signifient, indépendamment les uns des autres, H, F, CI, Br, CN, CF₃, OH, OCF₃, OCHF₂, SCH₃, SCF₃, phényle, O-phényle, COOH, COO-(C₁-C₆)-alkyle, CO-(C₁-C₆)-alkyle, (C₁-C₆)-alkyle, O-(C₁-C₆)-alkyle, OBn, SO₂-(C₁-C₄)-alkyle, SO₃H, SO₂NR9R10, NR9R10 ou SO₂-N-pipéridinyle, alkyle et phényle pouvant être monosubstitués ou polysubstitués par R12 ;
R7, R8 signifient H ;
m vaut 0, 1, 2, 3 ou 4 ;
R6 signifie OH, F, CI, Br, CN, OCH₃, OCF₃, CH₃, CF₃, (C₁-C₆)-alkyle ou O-(C₁-C₆)-alkyle, alkyle pouvant être monosubstitué ou polysubstitué par OH, F, CI, Br ou CN ;
R9, R10 signifient, indépendamment l'un de l'autre, H, (C₁-C₆)-alkyle ou phényle, alkyle pouvant être monosubstitué ou polysubstitué par F, Cl ou Br et phényle pouvant être monosubstitué ou polysubstitué par R6 ;
R11 signifie F, CI, Br, CN, OH, O-(C₁-C₆)-alkyle, (C₁-C₆)-alkyle, (C₂-C₆)-alcényle, (C₂-C₆)-alcynyle ou NR9R10 ;
R12 signifie F, CI, Br, CN, OH, O-(C₁-C₆)-alkyle, (C₁-C₆)-alkyle, (C₂-C₆)-alcényle, (C₂-C₆)-alcynyle, NR9R10, COOH, COO-(C₁-C₄)-alkyle, SCH₃, SCF₃, SO₂-(C₁-C₄)-alkyle, SO₃H ou SO₂NR9R10 ;
et leurs sels physiologiquement acceptables.

3. Composés selon la revendication 1 ou 2, **caractérisés en ce que**
R1 signifie H, F, CI, Br, CN, CF₃, OH, OCF₃, OCHF₂, SCH₃, SCF₃, phényle, O-phényle, COOH, COO-(C₁-C₆)-alkyle, CO-(C₁-C₆)-alkyle, (C₁-C₆)-alkyle, O-(C₁-C₆)-alkyle, OBn ;
R2 signifie H, F, CI, Br, CN, CF₃, OCF₃, OCHF₂, SCH₃, SCF₃, phényle, O-phényle, COOH, COO-(C₁-C₆)-alkyle, CO-(C₁-C₆)-alkyle, (C₁-C₆)-alkyle, O-(C₁-C₆)-alkyle, OBn ;
R3 signifie H, F, CI, Br, CN, CF₃, OH, OCF₃, OCHF₂, SCH₃, SCF₃, phényle, O-phényle, COOH, COO-(C₁-C₆)-alkyle, CO-(C₁-C₆)-alkyle, (C₁-C₆)-alkyle, O-(C₁-C₆)-alkyle, OBn ;
R4 signifie H, F, CI, Br, CN, CF₃, OCF₃, OCHF₂, SCH₃, SCF₃, phényle, O-phényle, COOH, COO-(C₁-C₆)-alkyle, CO-(C₁-C₆)-alkyle, (C₁-C₆)-alkyle, O-(C₁-C₆)-alkyle, OBn ;
R5 signifie H, F, CI, Br, CN, CF₃, OH, OCF₃, OCHF₂, SCH₃, SCF₃, phényle, O-phényle, COOH, COO-(C₁-C₆)-alkyle, CO-(C₁-C₆)-alkyle, (C₁-C₆)-alkyle, O-(C₁-C₆)-alkyle, OBn ;
au moins un des radicaux R1 à R5 ayant une signification différente d'hydrogène ;
R7, R8 signifient, indépendamment l'un de l'autre, H ou (C₁-C₆)-alkyle ; m vaut 0 ;
R9, R10 signifient, indépendamment l'un de l'autre, H ou (C₁-C₆)-alkyle, alkyle pouvant être monosubstitué ou polysubstitué par F, Cl ou Br ;
ainsi que leurs sels physiologiquement acceptables.

4. Composés selon l'une ou plusieurs des revendications 1 à 3 destinés à être utilisés comme médicament.

5. Médicament contenant un ou plusieurs des composés selon l'une ou plusieurs des revendications 1 à 3.

6. Médicament contenant un ou plusieurs des composés selon la revendication 5 et au moins une autre substance active.

7. Médicament selon la revendication 6, **caractérisé en ce qu'**il contient comme autre substance active un(e) ou plusieurs antidiabétiques, substances actives hypoglycémiantes, inhibiteurs de la HMGCoA-réductase, inhibiteurs de la résorption du cholestérol, agonistes de PPAR gamma, agonistes de PPAR alpha, agonistes de PPAR alpha/gamma, agonistes de PPAR delta, fibrates, inhibiteurs de MTP, inhibiteurs de la résorption de l'acide biliaire, inhibiteurs de CETP, adsorbants polymères de l'acide biliaire, inducteurs des récepteurs de LDL, inhibiteurs d'ACAT, antioxydants, inhibiteurs de l'ATP-citrate-lyase, inhibiteurs de la squalène synthétase, antagonistes de la lipoprotéine (a), agonistes du récepteur de HM74A, inhibiteurs de lipase, insulines, sulfonylurées, biguanides, méglitinides, thiazolidinediones, inhibiteurs de l'α-glucosidase, substances actives agissant sur le canal calcique dépendant de l'ATP des cellules bêta, inhibiteurs de la glycogène phosphorylase, antagonistes du récepteur du glucagon, activateurs de la glucokinase, inhibiteurs de la gluconéogenèse, inhibiteurs de la fructose-1,6-biphosphatase, modulateurs du transporteur 4 du glucose, inhibiteurs de la glutamine-fructose-6-phosphate-amidotransférase, inhibiteurs de la dipeptidylpeptidase-IV, inhibiteurs de la 11-bêta-hydroxystéroïde-déshydrogénase-1, inhibiteurs de la protéine-tyrosine-phosphatase-1B, modulateurs du transporteur 1 ou 2 du glucose dépendant du sodium, inhibiteurs de la lipase sensible aux hormones, inhibiteurs de l'acétyl-CoA carboxylase, inhibiteurs de la phosphoénolpyruvate-carboxykinase, inhibiteurs de la glycogène synthase kinase-3 bêta, inhibiteurs de la protéine kinase C bêta, antagonistes du récepteur de l'endothéline-A, inhibiteurs de la I kappa B kinase, modulateurs du récepteur des glucocorticoïdes, agonistes de MC4, agonistes de H3, agonistes de TNF, antagonistes de CRF BP, agonistes d'urocortine, antagonistes du récepteur de CB1, agonistes de MSH (hormone stimulant les mélanocytes), inhibiteurs de la réabsorption de la sérotonine, composés sérotoninergiques et noradrénergiques mixtes, agonistes de 5HT, agonistes de bombésine, agonistes de galanine, hormones de croissance, composés libérant l'hormone de croissance, agonistes de TRH, modulateurs de la protéine découplante 2 ou 3, agonistes de leptine, agonistes de DA, inhibiteurs de lipase/amylase, modulateurs de PPAR, modulateurs de RXR ou agonistes de TR-ß ou amphétamines.

8. Utilisation des composés selon l'une ou plusieurs des revendications 1 à 3 pour la préparation d'un médicament destiné à abaisser la glycémie.

9. Utilisation des composés selon l'une ou plusieurs des revendications 1 à 3 pour la préparation d'un médicament destiné au traitement du diabète.

10. Utilisation des selon l'une ou plusieurs des revendications 1 à 3 pour la préparation d'un médicament destiné à augmenter l'excrétion de l'insuline.

11. Procédé pour la préparation d'un médicament contenant un ou plusieurs des composés selon l'une ou plusieurs des revendications 1 à 3, **caractérisé en ce que** la substance active est mélangée avec un support pharmaceutiquement approprié et ce mélange est amené dans une forme appropriée pour l'administration.

12. Utilisation des composés selon l'une ou plusieurs des revendications 1 à 3 pour la préparation d'un médicament destiné au traitement de maladies du système nerveux central.

13. Utilisation des composés selon l'une ou plusieurs des revendications 1 à 3 pour la préparation d'un médicament destiné au traitement de la schizophrénie.

14. Utilisation des composés selon l'une ou plusieurs des revendications 1 à 3 pour la préparation d'un médicament destiné au traitement de la maladie d'Alzheimer.
